# EUROPÄISCHE PATENTANMELDUNG

(11) **EP 2 031 064 A1**
(43) Veröffentlichungstag der Anmeldung: **04.03.2009**
(21) Anmeldenummer: 07115227.6
(22) Anmeldetag: 29.08.2007
(51) Int. Cl.: C12N 15/13, C12N 15/85, C07K 16/00, C07K 14/505, C12N 9/72

(54) **Verfahren zur Steigerung von Proteintitern**

(71) Anmelder: Boehringer Ingelheim Pharma GmbH & Co. KG, 55216 Ingelheim (DE)
(72) Erfinder: Die Erfindernennung liegt noch nicht vor
(74) Vertreter: Hammann, Heinz

(57) **Zusammenfassung**

Die Erfindung betrifft Verfahren zur Steigerung des Titers eines Proteins von Interesse in einer Zelle sowie die verbesserte Produktion und Reinigung von optimierten Biomolekülen, deren Bestandteil u.a. die Domäne C_{H}3 ist. Ein oftmals beobachteter Effekt bei Biomolekülen ist die Abspaltung der C-terminalen Aminosäure(n) z.B. des C-terminalen Lysins. Die zumeist unvollständige Prozessierung beispielsweise der schweren Kette von Antikörpern führt zu Produktheterogenität. Zur Vermeidung dieser Produktheterogenität wurde mittels rekombinanter DNA Technologie das entsprechende Codon des C-terminalen Lysin der schweren Antikörperkette deletiert. Diese optimierten Antikörper führen zu einem, im Vergleich zum Wildtyp, erhöhten Produkttiter. Zusätzlich erweisen sie sich als vorteilhaft in der Reinigung durch ein besseres Elutionsverhalten aufgrund der reduzierten Ladungsheterogenität.

## Beschreibung

### HINTERGRUND DER ERFINDUNG

### TECHNISCHES GEBIET

Die Erfindung betrifft optimierte Proteine, insbesondere Antikörper Fc-Teile, bzw. Fc-Fusionsproteine und Verfahren zur Herstellung bzw. biopharmazeutischen Produktion solcher optimierten Antikörper und Fc-Fusionsproteine mit gesteigerter Produktivität sowie ein Verfahren zur Produktion und Aufreinigung von Proteinen, wobei das produzierte Biomolekül vollständig homogen bezüglich des C-terminalen Lysins ist.

### HINTERGRUND

Biomoleküle wie Proteine, Polynukleotide, Polysaccharide und dergleichen gewinnen als Medikamente, als Diagnostika, als Zusatzstoffe in Nahrungsmitteln, Waschmitteln und dergleichen, als Forschungsreagenzien und für viele weitere Anwendungen zunehmend an kommerzieller Bedeutung. Der Bedarf an solchen Biomolekülen lässt sich - z.B. im Falle von Proteinen - in aller Regel nicht mehr durch Isolierung der Moleküle aus natürlichen Quellen befriedigen sondern erfordert den Einsatz biotechnologischer Produktionsmethoden.

Die biotechnologische Herstellung von Proteinen beginnt typischerweise mit der Isolierung der DNA, die für das gewünschte Protein kodiert, und deren Klonierung in einen geeigneten Expressionsvektor. Nach Transfektion des rekombinanten Expressionsvektors in geeignete prokaryontische oder eukaryontische Expressionszellen und anschließender Selektion transfizierter, rekombinanter Zellen werden letztere in Fermentern kultiviert und das gewünschte Protein zur Expression gebracht. Anschließend erfolgt die Ernte der Zellen bzw. des Kulturüberstandes und die Aufarbeitung und Reinigung des darin enthaltenen Proteins.

Im Falle von Biopharmazeutika, wie etwa als Medikamente eingesetzten Proteinen, z.B. therapeutischen Antikörpern, ist die Produktausbeute entscheidend. Darüber hinaus ist auch die Abtrennung von Verunreinigungen von Bedeutung. Hierbei können prozess- und produktabhängige Verunreinigungen voneinander unterschieden werden. Die prozessabhängigen Verunreinigungen beinhalten Komponenten der Wirtszellen wie Proteine und Nukleinsäuren oder stammen aus der Zellkultur (wie Medienbestandteile) sowie aus der Aufarbeitung (wie etwa Salze oder abgelöste Chromatographie-Liganden). Darüber hinaus treten produktabhängige Verunreinigungen auf. Diese sind molekulare Varianten des Produkts mit abweichenden Eigenschaften. Hierzu zählen z.B. verkürzte Formen wie Vorläufer und hydrolytische Abbauprodukte, enzymatische Abspaltung von C-terminalen Aminosäureresten von Proteinen, aber auch modifizierte Formen, entstanden beispielsweise durch Desaminierungen, unterschiedliche Glykosylierungsmuster oder falsch verknüpfte Disulfidbrücken. Ebenso zu den produktabhängigen Varianten zählen Polymere und Aggregate. Als Kontaminanten werden alle weiteren Materialien chemischer, biochemischer oder mikrobiologischer Natur bezeichnet, die nicht direkt zum Herstellungsprozess gehören. Weitere Kontaminanten sind z.B. Viren, die unerwünschter Weise in Zellkulturen auftreten können.

Eine häufig beobachtet Produktvariante bei der Überexpression von rekombinanten Antikörpern oder Fc-Fusionsproteinen in Säugetierzellen zur Herstellung von neuen biopharmazeutischen Arzneimitteln beruht auf der Heterogenität am C-Terminus der schweren Kette von Immunoglobulinen durch enzymatische Abspaltung des C-terminalen Lysins. Zur exakten Beschreibung dieser Heterogenität müssen hoch auflösende analytische Methoden entwickelt werden. Für die Detektion und Quantifizierung der Ladungsheterogenität werden in der Qualitätskontrolle der pharmazeutischen Industrie folgende Methoden eingesetzt: Cation Ion Exchange Chromatography (CIEX), Isoelektrische Fokussierung (IEF) (nur Detektion), capillary Isoelectric Focusing (cIEF) sowie Liquid Chromatography Mass Spectrometry (LCMS). Jede produzierte Charge muss unter anderem bezüglich dieser Modifizierung bewertet und freigegeben werden.
Bei vielen der auf dem Markt befindlichen Moleküle dieser Klasse sind diese Produktheterogenitäten am C-Terminus der schweren Kette beschrieben. Dabei wird zwischen Antikörpermonomeren mit vollständig abgespaltenen Lysin (Lys0), mit einem abgespaltenen Lysin (Lys1) und ohne Lysinabspaltung (Lys2) am C-Terminus der schweren Kette unterschieden. Die verschiedenen unvollständig prozessierten Moleküle (Lys1 und Lys2) können bis zu 30% innerhalb einer Charge betragen (Santora et al. (1999) Analytical Biochemistry, 275(1): p. 98-108). Bei dem Herstellungsprozess von Remicade® (Infliximab) betrug die Heterogenität während der Fermentation ca. 20% (Lys0 und Lys1) und 80% (Lys2) (FDA, Product Review on Remicade, 1998). Weitere Beispiele für C-terminale Lysin-Prozessierung bei monoklonalen Antikörpern siehe Tabelle (Harris, R.J, (1995) Journal of Chromatography A, 705 (1), pp. 129-134).

| Protein | Aminosäure | Zelllinie/Quelle | Referenz |
|---|---|---|---|
| rCD4-IgG | Lys | transfizierte CHO | R.J. Harris, K.L. Wagner and M.W. Spellman, Eur. J. Biochem., 194 (1990) 611-620 |
| rhu MabHER2 | Lys | transfizierte CHO | R,J. Harris, A.A. Murnane, S.L. Utter, K.L, Wagner, E.T. Cox, G. Polastri, J.C. Helder and M.B. Sliwkowski, BiolTechnology, 11 (1993) 1293-1297 |
| OKT3 Mab | Lys | Hybridoma | P. Rao, A. Williams, A. Baldwin-Ferro, E. Hanigan, D. Kroon, M. Makowski, E. Meyer, V. Numsuwan, E. Rubin and A. Tran, BioPharm, 4 (1991) 38-43. |
| OKT3 Mab | Lys | Hybridoma | P. Rao, A. Williams, A. Baldwin-Ferro, E. Hanigan, D. Kroon, M. Makowski, E. Meyer, V. Numsuwan, E. Rubin and A. Tran, BioPharm, 4 (1991) 38-43. |
| CEM231 Mab | Lys | Hybridoma | J.P. McDonough, T.C. Furman, R.M. Bartholomew and R.A. Jue, US Pat. 5 126 250 (1992) |
| CEM231 Mab | Lys | Hybridoma | J.P. McDonough, T.C. Furman, R.M. Bartholomew and R.A. Jue, US Pat. 5 126 250 (1992) |
| Hu-anti-Tac Mab | Lys | transfizierte SP2/0 | D.A. Lewis, A.W. Guzzetta, W.S. Hancock and M. Costello, Anal. Chem., 66 (1994) 585-595. |
| 2-Chain tPA | Arg | transfizierte CHO | |
| 2-Chain tPA | Arg | Melanoma | |
| hu EPO | Arg | humanes Urin | M.A. Recny, H.A. Scoble and Y. Kim, J. Biol. Chem., 262 (1987) 17156-17163 |
| rhu EPO | Arg | transfizierte CHO | M.A. Recny, H.A. Scoble and Y. Kim, J. Biol. Chem., 262 (1987) 17156-17163 |
| Quelle: Harris, R.J. (1995) Journal of Chromatography A, 705 (1), pp. 129-134 | | | |

Die Ursache für diese Produktheterogenität ist bisher nicht bekannt. Es ist unklar, ob die Struktur der Kette, die Wirtszelle oder die Fermentationsbedingungen und damit unterschiedliche metabolische Prozesse in der Zelle einen wesentlichen Einfluss nehmen. Weiterhin ist bisher unbekannt, an welche Stelle der Produktherstellung in der Zelle (co-translational, post-translational), an welchem Ort und durch welche Carboxypetidase die Abspaltung des Lysins erfolgt. Mögliche Chargenschwankungen können somit nicht verhindert werden und Gegensteuerung ist somit nicht gezielt möglich.

Produktheterogenitäten können auch durch andere C-terminale AminosäureDeletionen verursacht werden, wie z. B. durch eine Deletion des C-terminalen Arginins bei den Proteinen tPA oder EPO (Harris, R.J, (1995) Journal of Chromatography A, 705 (1), pp. 129-134).

Als Ausgangspunkt für die Bewertung der Produktionscharge dienen die physikochemikalischen Produkteigenschaften, die Reinheit, die Homogenität sowie die Wirksamkeit und Sicherheit des Produkts.
Elektrophoretische (IEF) bzw. chromatographische (IEC, SEC, RP) Trennverfahren sowie massenspektroskopische Verfahren (MS, ESI, MALDI) dienen zur Bewertung der Produktreinheit und Produktheterogenität .
Die Kontrolle der Produktreinheit stellt die ausreichende Abreicherung von Verunreinigungen und die Entfernung von im Herstellungsverfahren durch enzymatische, mechanische oder chemische Vorgänge entstandenen Spaltprodukten und aggregierten Proteinmolekülen sicher. Die Bewertung der Produkthomogenität erfolgt primär anhand der Abweichungen im Glykosylierungsmuster und der Ladungsheterogenität. Die Wirksamkeit eines Produktes beschreibt dessen biologische Aktivität, die sich bei Antikörpern aus Eigenschaften wie das Antigenbindungsvermögen, die Induktion von Effektorfunktionen, Serum-Halbwertszeit u.a. zusammensetzt. Bestimmende Faktoren für die Produktsicherheit sind u.a. die Sterilität und die bakterielle Endotoxinbelastung der Produktcharge.

Aufgrund der Vielzahl an Kontrollwerten, die bei einer erzeugten Produktionscharge für dessen Freigabe gewährleistet bzw. eingehalten werden müssen, ist eine Reduktion der Kontrollwerte, z. B. durch Eliminierung des die Charge beeinflussenden Parameters, erstrebenswert.

Weiterhin ist bei der biotechnologischen Herstellung von Proteinen ein hoher Produkttiter und eine hohe spezifische Produktivität der Zellen wünschenswert.

Es ergibt sich somit die Aufgabe, einen verbesserten Herstellprozess bereit zu stellen. Hinsichtlich der Produktexpression, der Produktreinigung und Produktstabilität dürfen bei der Herstellung keine negativen Einflüsse auftreten.

Die vorliegende Erfindung löst diese Aufgabe überraschend durch ein Verfahren zur Herstellung von Proteinen, welches eine gesteigerte Ausbeute ermöglicht, indem bereits auf DNA-Ebene das C-terminal kodierende Kodon (z.B. Lysin) entfernt und anschließend ein Stopp-Kodon eingefügt wird. Dieses Verfahren ermöglicht eine Steigerung des Proteintiters, insbesondere von Antikörpern, die eine C-terminale Lysin-Deletion an den schweren Ketten aufweisen.

### ZUSAMMENFASSUNG DER ERFINDUNG

Die vorliegende Erfindung beschreibt rekombinante DNA Konstrukte von Proteinen, insbesondere von Antikörpermolekülen wie IgG1, IgG2, IgG3, IgG4 und Fc-Fusionskonstrukte, die eine Deletion des C-terminalen Lysins aufweisen. Durch die durchgeführte Änderung des Expressionskonstruktes und die Deletion des C-terminalen Lys-Kodons werden nur noch Moleküle mit einem homogen C-Terminus der schweren Kette hergestellt.

Bei der Überexpression von beispielsweise rekombinanten Antikörpern oder Fc-Fusionsproteinen in Säugetierzellen zur Herstellung von neuen biopharmazeutischen Arzneimittel treten häufig Moleküle auf, die am C-Terminus der schweren Kette Heterogenitäten aufweisen. Das gereinigte Endprodukt weist bezüglich des C-Terminus der schweren Kette drei unterschiedliche Spezies auf: 1) vollständige Ketten mit C-terminalem Lysin gemäß der DNA Sequenz (Lys 2) oder 2) unvollständige Kette (Lys 1) und 3) Deletion des C-terminalen Lysins an beiden Ketten (Lys 0). Die Anteile der beiden Spezies sind nicht vorhersagbar. Somit können Unterschiede je nach Zelle, Fermentationsbedingungen und Herstellcharge auftreten. Es ist unklar, ob die Antikörperstruktur einen Einfluss auf diese intrazelluläre enzymatische Abspaltung des Lysins hat.
Bei den bisher auf dem Markt befindlichen Molekülen wurde derart verfahren, dass die komplette DNA-Sequenz der schweren Kette exprimiert wird und auftretende Heterogenitäten am C- Terminus mit hohem Aufwand analysiert und dokumentiert werden. Zur Charakterisierung des Produktes müssen somit exakte Methoden zur Analyse des C-Terminus entwickelt werden und alle Chargen bezüglich dieses Merkmals analysiert werden (Alexandru C. Lazar et al; Rapid Commun. Mass Spectrum. 2004; 18: 239-244, Lintao Wang et al; Pharmaceutical Research, Vol.22, No.8, 2005). Der Aufwand zur Analyse der auftretenden Produktheterogenitäten ist somit hoch. Eine Reduktion des Analysenaufwandes und der Analysekosten wäre wünschenswert.

Die Ursache der beschriebenen Produktheterogenität ist bisher nicht bekannt. Es ist unklar, ob die Struktur der Kette, die Wirtszelle oder die Fermentationsbedingungen und damit unterschiedliche metabolische Prozesse in der Zelle einen wesentlichen Einfluss nehmen. Weiterhin ist bisher unbekannt, an welcher Stelle der Produktherstellung in der Zelle (co-translational, post-translational), an welchem Ort und durch welches Enzym die Abspaltung des Lysins erfolgt. Mögliche Chargenschwankungen können somit nicht verhindert werden und Gegensteuerung ist somit nicht gezielt möglich.

Es gibt bisher keinen Hinweis, dass bei der Applikation dieser Produkte aufgrund der Heterogenität im C-Terminus nachteilige Effekte, wie z.B. immunopathologische Nebenwirkungen, auftreten. Daher scheint auch die nicht native Sequenz ohne C-terminales Lysin hinsichtlich der klinischen Wirksamkeit und Verträglichkeit unbedenklich und der nativen Sequenz gleichwertig zu sein.

Bisher sind jedoch keine Konstrukte für therapeutische Proteine, insbesondere Antikörper oder Fc-Fusionskonstrukte, mit der Deletion des C-terminalen Lys-Kodons beschrieben, denn das C-terminale Lysin in der schweren Kette von IgGs ist hoch konserviert.
Abweichend von dem Stand der Technik wurde bei der vorliegenden Erfindung bereits auf DNA-Ebene am 3' Ende das Kodon für Lysin beim Expressionskonstrukt für die schwere Kette von Antikörpern deletiert. Bei allen IgG Subtypen ist der C-Terminus der schweren Kette sehr konserviert und das Lysin am C-Terminus ist zum Beispiel sowohl bei humanen als auch Maus Antikörpern immer vorhanden. Aufgrund dieses Sachverhaltes ist zu erwarten, dass das Lysin für die Expression, Faltung oder Sekretion eine wesentliche Bedeutung hat. Überraschender Weise konnte in unseren Experimenten mit unterschiedlichen IgG Klassen jedoch erstmalig gezeigt werden, dass trotz Deletion des C-terminalen Lysins die Moleküle in tierischen Zellkultursystemen exprimiert werden und die native Proteinstruktur ins Medium sezerniert wird. Besonders überraschend hierbei ist die beobachtete und vollkommen unerwartete Steigerung der Produkttiter beim Einsatz dieser Konstrukte. Dies ist im Hinblick auf die hohe Konservierung der C-terminalen Lysin-Position in den bevorzugt humanen Immunoglobulinen unerwartet. Der Produkttiter ist beim Einsatz dieser Expressionskonstrukte um mindestens 10 %, bevorzugt mindestens 20% und besonders bevorzugt um mindestens 50% gesteigert.

Die Vermeidung der Produktheterogenität durch die Lys-Kodondeletion konnte zudem durch die Analyse produzierter Antikörper-Isotypen qualitativ und quantitativ belegt werden. Für zwei verschiedene Isotypen (IgG1 und IgG4) wurden zum Vergleich der Wildtyp-Antikörper und die entsprechende Lysin-Deletionsmutante exprimiert und gereinigt. Im Anschluss erfolgte die Proteincharakterisierung. Darüber hinaus konnte entgegen den Erwartungen gezeigt werden, dass die Produkttiter um mindestens 10-20% gesteigert werden konnte. Für die Aufarbeitung des Produktes (Protein A Affinitätschromatographie) und die Proteincharakterisierung wurden keine nachteiligen Effekte der Lysin-Kodondeletion festgestellt. Die weitere Analyse des Reinigungsverfahrens und der Produktcharakterisierung (Produktausbeute, Aggregationsverhalten) ergab ebenfalls keinen nachteiligen Einfluss der Lys-Deletionsmutanten. Aufgrund der höheren Robustheit für den Herstellprozess, der Reduktion des analytischen Aufwandes und der gesteigerten Produkttiter ist dieses neue Verfahren dem bisherigem Stand der Technik deutlich überlegen.

Der wesentliche Vorteil zum jetzigen Stand der Technik ist, dass unter Verwendung dieser Konstrukte nur die Variante des C-Terminus ohne Lysin für die schweren Kette auftreten kann. Somit entfällt eine Möglichkeit für Chargenschwankungen und der Anteil an Produktcharakterisierungsarbeiten. Besonders überraschend ist bei der vorliegenden Erfindung, dass die Konstrukte ohne C-terminales Lysin zu gesteigerten Produkttitern führen, was für eine hohe Ausbeute besonders vorteilhaft ist.

Die vorliegende Erfindung lässt sich bevorzugt auf Prozesse zur Herstellung von rekombinanten Antikörpern und/oder Fc-Fusionsproteinen anwenden. Die vorliegende Erfindung lässt sich jedoch auch auf andere Moleküle anwenden, die C-terminale Aminosäuredeletionen aufweisen. Beispiele hierfür sind EPO und tPA, bei denen C-terminale Arginin-Deletionen auftreten.

Die Erfindung betrifft die verbesserte Produktion und Reinigung von optimierten Proteinen, deren Bestandteil u.a. die Immunoglobulin-Domäne C_{H}3 ist. Ein oftmals beobachteter Effekt bei diesen Proteinen ist die Abspaltung des C-terminalen Lysins. Diese zumeist unvollständige Prozessierung der schweren Kette führt zu Produktheterogenität. Zur Vermeidung dieser Produktheterogenität wurde mittels rekombinanter DNA Technologie das entsprechende Kodon des C-terminalen Lysins der schweren Antikörperkette deletiert. Diese Deletion im optimierten Antikörper führt überraschender Weise nicht zu einem Nachteil bei der Expression bzw. intrazellulären Proteinprozessierung sondern zu einem, im Vergleich zum Wildtyp, erhöhten Produkttiter. Zusätzlich erweisen sich die optimierten Antikörper als vorteilhaft in der Reinigung durch ein besseres Elutionsverhalten aufgrund der reduzierten Ladungsheterogenität und sie zeichnen sich durch eine verbesserte Homogenität aus. Zudem ist vorteilhaft, dass bei der Reinigung des Proteins von Interesse eine niedrigere Salzkonzentration zum Einsatz kommt im Vergleich zur Aufreinigung eines Proteins ohne die Deletion der C-terminalen Aminosäure.

Die vorliegende Erfindung ergibt sich nicht aus dem Stand der Technik. Zum gegenwärtigen Zeitpunkt muss die Produktheterogenität für jede Produktionscharge analysiert werden bevor eine Freigabe erfolgen kann. Für die qualitative und quantitative Bestimmung der Heterogenität von Lysinresten am C-Terminus der schweren Kette müssen arbeits- und kostenintensive Analysemethoden eingesetzt werden.
Als etablierte Methoden zur quantitativen Bestimmung der Antikörperisoformen werden in der Qualitätskontrolle der pharmazeutischen Industrie säulenchromatographische Trennmethoden (schwache Kationentauscher, WCX) z.T. in Kombination mit Massenspektroskopie (LC-MS) oder elektrophoretische Trennmethoden eingesetzt (kapillare Isoelektrische Fokussierung, CIEF). Die gelisoelektrophoretische Fokussierung erlaubt lediglich eine qualitative Bewertung der Lysin-Heterogenität.

Ein Ansatz zur Reduzierung der Ladungsheterogenität durch C-terminale Lysinreste der schweren Antikörperkette ist in bestehenden Verfahren zur Verringerung der Heterogenität von monoklonalen Antikörpern beschrieben (EP0361902, US5126250). Eine Reduzierung der Heterogenität wird hier durch verschiedenen Methoden bewirkt, wie der pH-Wertabsenkung, der enzymatischen Abspaltung C-terminaler Lysinreste durch Carboxypeptidase oder die Zugabe von Ascitesflüssigkeit.

Bei dem enzymatischen Verfahren erfolgt die Reduktion der Ladungsheterogenität über die Abspaltung von C-terminalen Lysinresten der schweren Kette von Immunoglobulin Antikörpern mittels des Enzyms Carboxypeptidase. Dieses Verfahren erreicht allerdings nur eine Umwandlung von 95% der Antikörper in die homogene Antikörperform (Lys0). Weitere Verfahren bestehen in der Inkubation der heterogenen Antikörperformen mit Ascitesflüssigkeit in verschiedenen Verhältnissen (2:1 bis 1:10) oder in der pH-Wert Absenkung des Kulturmediums. Die Effizienz dieser Verfahren zur C-terminalen Lysin Abspaltung beträgt ebenfalls nur ca. 95%. Alle Verfahren sind zudem zeitaufwändig (>24h).

### BESCHREIBUNG DER ABBILDUNGEN

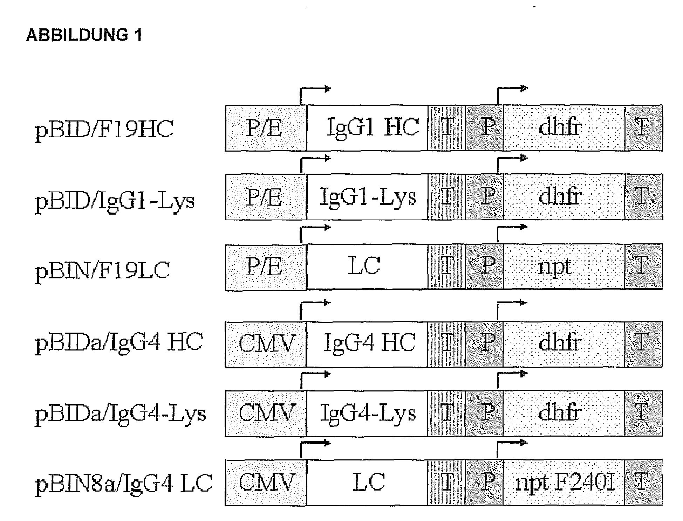

### ABBILDUNG 1: SCHEMATISCHE DARSTELLUNG DER REKOMBINANTEN VEKTOREN

Die hier dargestellten Vektoren werden zur Expression der monoklonalen Antikörper vom IgG1- und IgG4-Isotyp in CHO-DG44 Zellen eingesetzt. Bei "P/E" handelt es sich um eine Kombination aus CMV-Enhancer und Hamster Ub/S27a-Promotor, bei "CMV" um eine Kombination aus CMV-Enhancer und -Promotor, bei "P" lediglich um ein Promotorelement und bei "T" um ein Terminationssignal für die Transkription, das zur Polyadenylierung der transkribierten mRNA benötigt wird. Die Position und Richtung der Transkriptionsinitiation innerhalb jeder Transkriptionseinheit wird durch einen Pfeil angezeigt. Der amplifizierbare Selektionsmarker Dlhydrofolat-Reduktase ist mit "dhfr" abgekürzt. Der Selektionsmarker Neomycin-Phosphotransferase ist mit "npt" gekennzeichnet und die durch Punktmutation F2401 erzeugte Neomycin-Phosphotransferase-Mutante mit "npt F240I". "IgG1 HC" kodiert für die schwere Kette des Wildtyp F19-Antikörpers vom IgG1 Isotyp und "IgG1-Lys" für die schwere Kette dieses Antikörpers mit einer C-terminalen Lysin-Deletion. "IgG4 HC" kennzeichnet das Gene für die schwere Kette des IgG4-Wildtyps und "IgG4-Lys" wiederum die schwere Kette des IgG4s mit einer C-terminalen Lysin-Deletion. "LC" kodiert für die leichte Kette des IgG1- bzw. IgG4-Antikörpers.

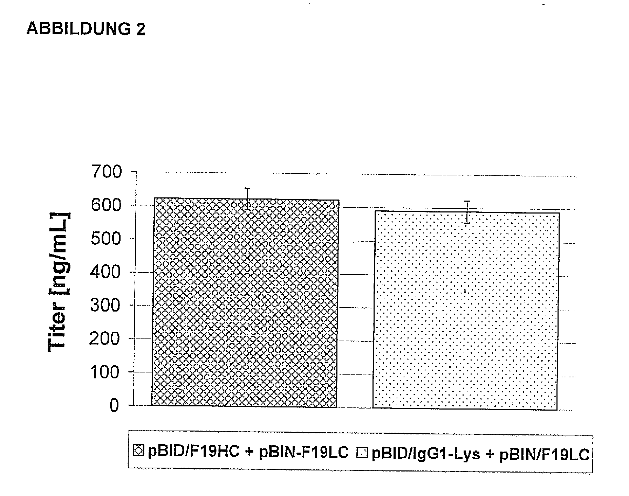

### ABBILDUNG 2: EINFLUSS DER C-TERMINALEN LYSIN-DELETION AUF DIE TRANSIENTE EXPRESSION EINES IGG1-ANTIKÖRPERS

Zur Prüfung, ob das konservierte C-terminale Lysin der schweren Kette einen Einfluss auf die Expression oder Sekretion des IgG1 Moleküls hat, wird eine Co-Transfektion von CHO-DG44 Zellen mit den Plasmidkombinationen pBID/F19HC und pBIN/F19LC (IgG1 mit C-terminalem Lysin, karierter Balken) bzw. BID/IgG1-Lys und pBIN/F19LC (IgG1 mit C-terminaler Lysin-Deletion, gepunkteter Balken) durchgeführt. Gleichzeitig wird ein SEAP-Expressionsplasmid (= sezernierte alkalische Phosphatase) co-transfiziert, um einen Abgleich der Transfektionseffizienz vorzunehmen. 48 h nach Transfektion werden die Zellkulturüberstände abgenommen und die IgG1-Titer mittels ELISA sowie die SEAP-Aktivität bestimmt. Der IgG1-Titer wird hinsichtlich der Transfektionseffizienz korrigiert. Die Abbildung zeigt den Mittelwert aus jeweils 10 Parallelpools mit vergleichbaren Produktmengen für beide Varianten.

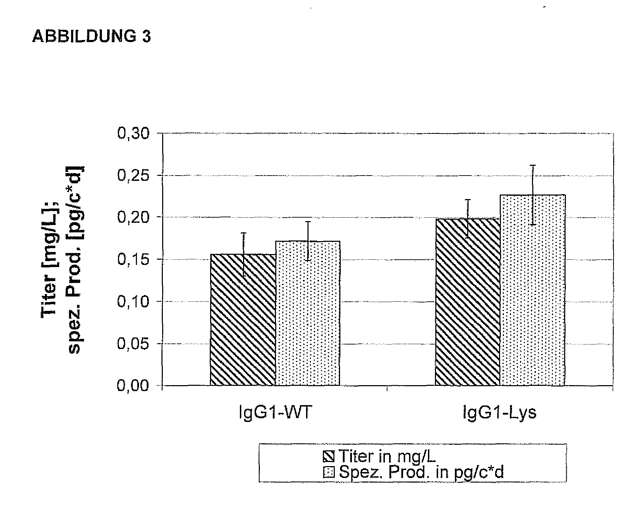

### ABBILDUNG 3: EXPRESSION VON IGG1-WILDTYP UND IGG1-LYSIN-DELETIONSMUTANTE IN STABILEN UNAMPLIFIZIERTEN ZELLPOOLS

In stabil transfizierten Zellen wird der Einfluss der C-terminalen Lysin-Deletion auf die Expression eines IgG1 Antikörpers untersucht. Dazu werden CHO-DG44 Zellen mit den Plasmidkombinationen pBID/F19HC und pBIN/F19LC (IgG1 mit C-terminalem Lysin = IgG1-WT) bzw. BID/IgG1-Lys und pBIN/F19LC (IgG1 mit C-terminaler Lysin-Deletion = IgG1-Lys) transfiziert. Nach einer zwei- bis dreiwöchigen Selektion der transfizierten Zellpools, jeweils 10 pro Plasmidkombination, in Hypoxanthin /Thymidin-freiem Medium mit Zusatz von G418 wird die Konzentration des produzierten IgG1 Antikörpers im Zellkulturüberstand durch ELISA ermittelt und die spezifische Produktivität pro Zelle und Tag berechnet. Die Balken repräsentieren die Mittelwerte der spezifischen Produktivität (gepunktete Balken) bzw. des Titers (gestreifte Balken) aller Pools im Test aus jeweils 3 - 4 Kultivierungspassagen in 75cm² Zellkulturflaschen.

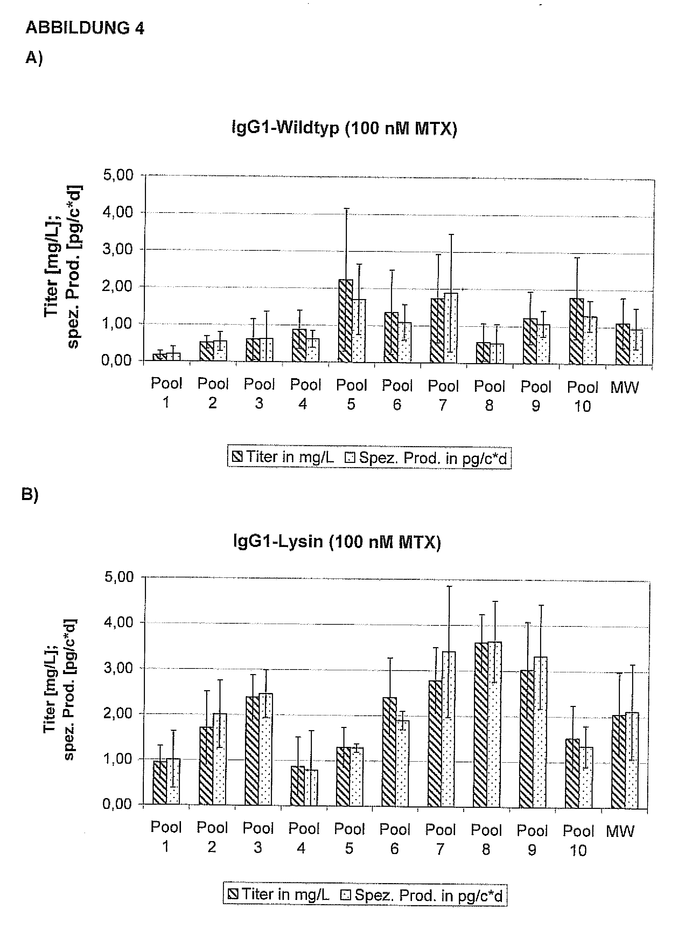

### ABBILDUNG 4: EXPRESSION VON IGG1-WILDTYP UND IGG1-LYSIN-DELETIONSMUTANTE IN STABILEN AMPLIFIZIERTEN ZELLPOOLS

CHO-DG44 Zellen werden mit den Plasmidkombinationen pBID/F19HC und pBIN/F19LC (IgG1 mit C-terminalem Lysin = IgG1-Wildtyp) bzw. BID/IgG1-Lys und pBIN/F19LC (IgG1 mit C-terminaler Lysin-Deletion = IgG1-Lysin) transfiziert. Nach einer zwei- bis dreiwöchigen Selektion der transfizierten Zellpools (jeweils 10 Pools pro Plasmidkombination) in Hypoxanthin /Thymidin-freiem Medium mit Zusatz von G418 wird nachfolgend eine DHFR-vermittelte Genamplifikation durch Zusatz von 100 nM Methotrexat (MTX) zum Kultivierungsmedium durchgeführt. Die Konzentration des produzierten IgG1 Antikörpers im Zellkulturüberstand wird durch ELISA ermittelt und die spezifische Produktivität pro Zelle und Tag berechnet. Die Balken repräsentieren zum einen die Mittelwerte der spezifischen Produktivität (gepunktete Balken) bzw. des Titers (gestreifte Balken) jedes einzelnen Pools im Test aus jeweils 6 Kultivierungspassagen in 75cm² Zellkulturflaschen. Zum anderen ist auch der Mittelwert (MW) aus allen Pooldaten angegeben.
In Abbildung 4A sind die Daten der mit dem IgG1-Wildtyp transfizierten Zellpools dargestellt, in Abbildung 4B die Daten der mit der IgG1-Lysin-Deletionsvariante transfizierten Zellpools. Letztere produzieren im Durchschnitt 86 % mehr Antikörper bei 120 % höherer spezifischer Produktivität als die mit dem IgG1-Wildtyp transfizierten Zellpools.

### ABBILDUNG 5: EINFLUSS DER C-TERMINALEN LYSIN-DELETION AUF DIE TRANSIENTE EXPRESSION EINES IGG4-ANTIKÖRPERS

Zur Prüfung, ob das konservierte C-terminale Lysin der schweren Kette einen Einfluss auf die Expression oder Sekretion des IgG4 Moleküls hat, wird eine Co-Transfektion von CHO-DG44 Zellen mit den Plasmidkombinationen pBIDa/IgG4 HC und pBIN8a/IgG4 LC (IgG4 mit C-terminalem Lysin, karierter Balken) bzw. BIDa/IgG4-Lys und pBIN8a/IgG4 LC (IgG4 mit C-terminaler Lysin-Deletion, gepunkteter Balken) durchgeführt. Gleichzeitig wird ein SEAP-Expressionsplasmid (= sezernierte alkalische Phosphatase) co-transfiziert, um einen Abgleich der Transfektionseffizienz vorzunehmen. 48 h nach Transfektion werden die Zellkulturüberstände abgenommen und die IgG4-Titer mittels ELISA sowie die SEAP-Aktivität bestimmt. Der IgG4-Titer wird hinsichtlich der Transfektionseffizienz korrigiert. Die Abbildung zeigt den Mittelwert aus jeweils 10 Parallelpools mit sogar etwas höhereren Produkttitern des IgG4-Antikörpers mit C-terminaler Lysin-Deletion.

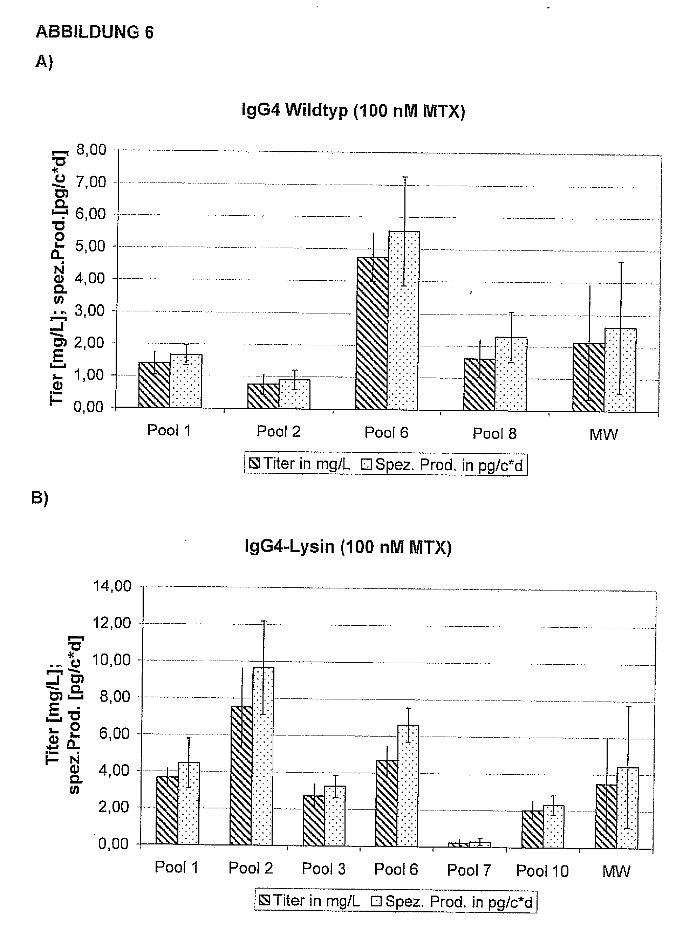

### ABBILDUNG 6: EXPRESSION VON IGG4-WILDTYP UND IGG4-LYSIN-DELETIONSMUTANTE IN STABILEN AMPLIFIZIERTEN ZELLPOOLS

CHO-DG44 Zellen werden mit den Plasmidkombinationen pBIDa/IgG4 HC und pBIN8a/IgG4 LC (IgG4 mit C-terminalem Lysin = IgG4-Wildtyp) bzw. BIDa/IgG4-Lys und pBIN8a/IgG4 LC (IgG4 mit C-terminaler Lysin-Deletion = IgG4-Lysin) transfiziert. Nach einer zwei- bis dreiwöchigen Selektion der transfizierten Zellpools (jeweils 10 Pools pro Plasmidkombination) in Hypoxanthin /Thymidin-freiem Medium mit Zusatz von G418 wird nachfolgend eine DHFR-vermittelte Genamplifikation durch Zusatz von 100 nM Methotrexat (MTX) zum Kultivierungsmedium durchgeführt, wobei hier für den IgG4-Wildtyp 4 und für die IgG4-Lysin-Deletionsvariante 6 erfolgreich amplifizierte Zellpools erhalten werden. Die Konzentration des produzierten IgG4 Antikörpers im Zellkulturüberstand wird durch ELISA ermittelt und die spezifische Produktivität pro Zelle und Tag berechnet. Die Balken repräsentieren zum einen die Mittelwerte der spezifischen Produktivität (gepunktete Balken) bzw. des Titers (gestreifte Balken) jedes einzelnen Pools im Test aus jeweils 6 Kultivierungspassagen in 75cm² Zellkulturflaschen. Zum anderen ist auch der Mittelwert (MW) aus allen Pooldaten angegeben.
In Abbildung 6A sind die Daten der mit dem IgG4-Wildtyp transfizierten Zellpools dargestellt, in Abbildung 6B die Daten der mit der IgG4-Lysin-Deletionsvariante transfizierten Zellpools. Letztere produzieren im Durchschnitt 63 % mehr Antikörper bei 70 % höherer spezifischer Produktivität als die mit dem IgG4-Wildtyp transfizierten Zellpools.

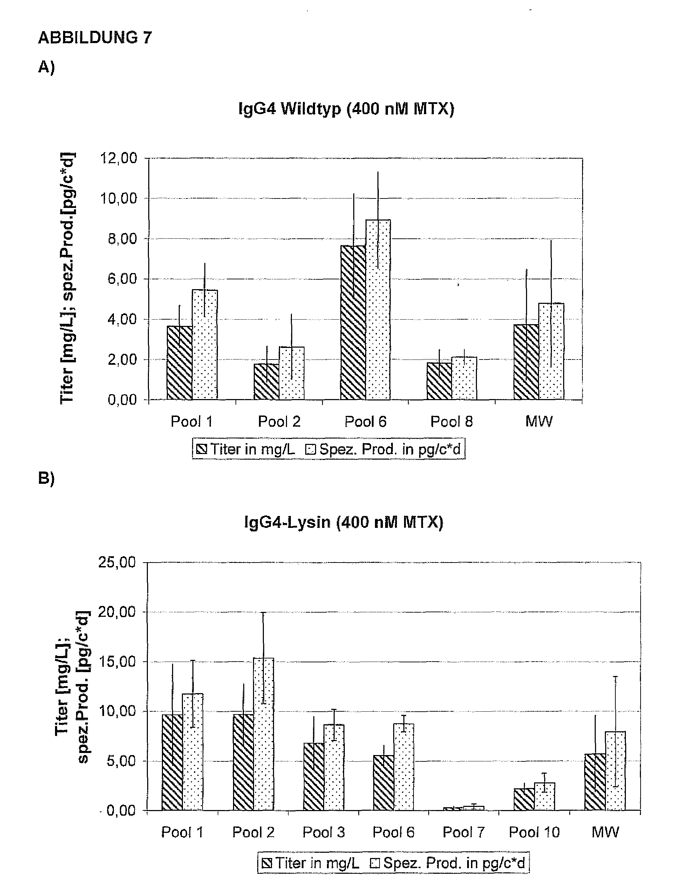

### ABBILDUNG 7: EXPRESSION VON IGG4-WILDTYP UND IGG4-LYSIN-DELETIONSMUTANTE IN ZELLPOOLS NACH ZWEITER GENAMPLIFIKATIONSRUNDE

CHO-DG44 Zellen werden mit den Plasmidkombinationen pBIDa/IgG4 HC und pBIN8a/IgG4 LC (IgG4 mit C-terminalem Lysin = IgG4-Wildtyp) bzw. BIDa/IgG4-Lys und pBIN8a/IgG4 LC (IgG4 mit C-terminaler Lysin-Deletion = IgG4-Lysin) transfiziert. Zunächst erfolgt eine zwei- bis dreiwöchigen Selektion der transfizierten Zellpools (jeweils 10 Pools pro Plasmidkombination) in Hypoxanthin /Thymidin-freiem Medium mit Zusatz von G418. Nachfolgend wird eine stufenweise DHFR-vermittelte Genamplifikation durchgeführt. Im ersten Schritt wird dem Kultivierungsmedium 100 nM Methotrexat (MTX) zugesetzt. Mit diesen aus dieser Genamplifikation hervorgegangen stabilen Zellpools wird eine zweite Genamplifikationsrunde durch Zusatz von 400 nM MTX zum Kultivierungssmedium durchgeführt. Hierbei werden für den IgG4-Wildtyp 4 und für die IgG4-Lysin-Deletionsvariante 6 erfolgreich amplifizierte Zellpools erhalten. Die Konzentration des produzierten IgG4 Antikörpers im Zellkulturüberstand wird durch ELISA ermittelt und die spezifische Produktivität pro Zelle und Tag berechnet. Die Balken repräsentieren zum einen die Mittelwerte der spezifischen Produktivität (gepunktete Balken) bzw. des Titers (gestreifte Balken) jedes einzelnen Pools im Test aus jeweils 4 Kultivierungspassagen in 75cm² Zellkulturflaschen. Zum anderen ist auch der Mittelwert (MW) aus allen Pooldaten angegeben.
In Abbildung 7A sind die Daten der mit dem IgG4-Wildtyp transfizierten Zellpools dargestellt, in Abbildung 7B die Daten der mit der IgG4-Lysin-Deletionsvariante transfizierten Zellpools. Letztere produzieren im Durchschnitt 53 % mehr Antikörper bei 66 % höherer spezifischer Produktivität als die mit dem IgG4-Wildtyp transfizierten Zellpools.

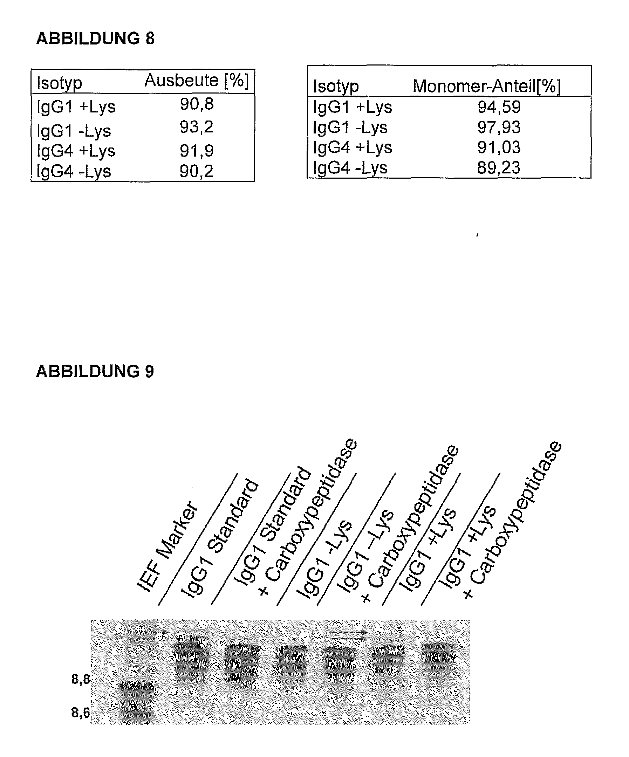

### ABBILDUNG 8: QUANTIFIZIERUNG DER PRODUKTAUSBEUTE MITTELS PROTEIN A HPLC

Die ermittelten Werte für die Produktausbeute von IgG1 und IgG4 liegen unabhängig von der Lysindeletion bei über 90%. Der Monomeranteil der Isotypen und der entsprechenden Lysin-Deletionsvarianten liegt im Bereich von 89,23 bis 97,93 %.

Sowohl die Ausbeute als auch der Monomerengehalt sind beim IgG1 -Lys höher als bei der WT-Variante.

### ABBILDUNG 9: ISOELEKTRISCHE FOKUSSIERUNG (IEF) DER ISOTYPEN IGG1 UND IGG4

Die Antikörper wurden *in vitro* mit Carboxypeptidase B inkubiert um vorhandenes C-terminales Lysin abzuspalten. Der Isotyp IgG1 (+Lysin ) (= IgG1-Wildtyp) weist nach der Inkubation mit Carboxypeptidase B eine geringere Anzahl an Proteinbanden auf (Abspaltung der C-terminalen Lysine bei Lys2 und Lys1 => Lys0). Der Isotyp IgG1 (-Lysin) (= C-terminale Lysindeletionsvariante) weist ein identisches Bandenmuster unabhängig von der Carboxypeptidase B Inkubation auf. IEF Markerbanden finden sich bei 8.8 kDa und 8.6 kDa.

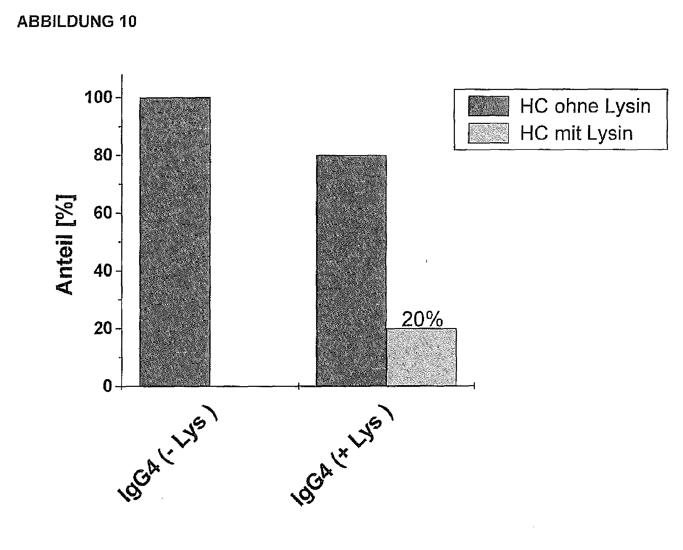

### ABBILDUNG 10: NACHWEIS DES C-TERMINALEN LYSINS MITTELS LC-MS

Für den Isotyp IgG4 (+Lysin) (= IgG4-Wildtyp) liegt der Anteil an schwerer Kette (HC) mit C-terminalem Lysin bei 20% (hellgrauer Balken HC mit Lysin), bei der Variante IgG4 (-Lysin) (=C-terminale Lysindeletionsvariante) bei 0%. Die dunkelgrauen Balken repräsentieren die Anteile an schweren Ketten (HC) ohne Lysin.

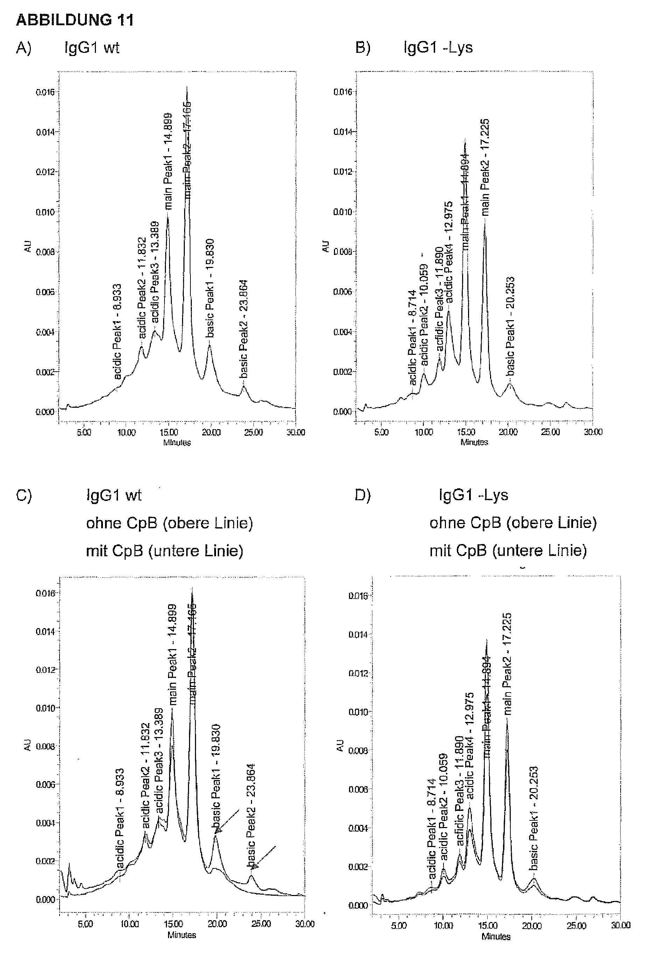

### ABBILDUNG 11: AUFTRENNUNG DER ANTIKÖRPER MITTELS WCX

Auftrennung des IgG1-WT (A) und IgG1 -Lysin (B) mittles weak cationic exchange (WCX). Die enzymatische Lysinabspaltung mittels Carboxypeptidase B zeigt bei dem WT IgG1 eine Reduktion der basischen Peaks 1 und 2. Der Overlay (C) von IgG1 WT ohne CpB (obere Linie) und IgG1 WT +CpB (untere Linie) zeigt die Reduktion der basischen Peakflächen um insgesamt 9,8%. Der Overlay (D) von IgG1-Lys ohne CpB (obere Linie) und IgG1-Lys +CpB (untere Linie) zeigt keine Reduktion der basischen Peakfläche (∼unter 1 %).

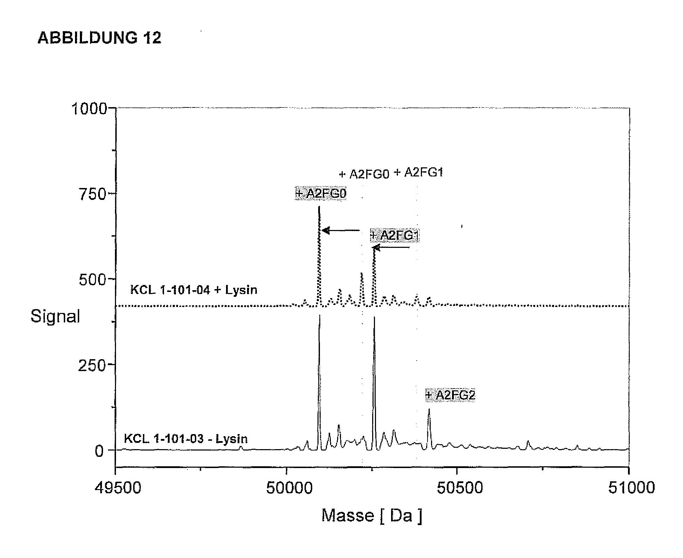

### ABBILDUNG 12: QUANTIFIZIERUNG DES C-TERMINALEN LYSINS MITTELS LC-MS

Quantifizierung des C-terminalen Lysinanteils an der schweren Antikörperkette von IgG4 mittels LC-MS (IgG4 WT: gepunktete (obere) Linie und IgG4-Lys: durchgezogenen (untere) Linie). Nach Reduktion und chromatographischer Trennung wurde der quantitative Anteil der schweren Kette mit C-terminalen Lysin (HC 1 - 447 mit Lysin) bzw. ohne Lysin (HC 1 - 446 ohne Lysin) bestimmt. Die Pfeile kennzeichnen den durch die Lysinabspaltung verursachten Massenshift in Abhängigkeit vom Glykosylierungszustand. Markierte Peaks kennzeichnen Glykosylierungen der schweren Kette (schwarz: HC mit C-terminalen Lysin, grau hinterlegt: HC ohne C-terminales Lysin).

### DETAILLIERTE BESCHREIBUNG DER ERFINDUNG

### DEFINITIONEN

Im Rahmen dieser Erfindungsbeschreibung verwendete Begriffe und Bezeichnungen haben folgende im Anschluss definierte Bedeutungen. Die allgemeinen Ausführungsformen "enthaltend" oder "enthält" schließt die speziellere Ausführungsform "bestehend aus" mit ein. Ferner werden "Einzahl" und "Mehrzahl" nicht begrenzend verwendet.

Der Begriff "Titer" ist eine Angabe der Produktkonzentration in einem definiertem Volumen, z.B. ng/mL, mg/mL, mg/L, g/L.

Mit dem Begriff "spezifische Produktivität" ist die von der Zelle produzierte Menge an Protein in pg pro Zelle und Tag gemeint. Sie wird mit der Formel pg/((Ct-Co) t / In (Ct-Co)) berechnet, wobei Co und Ct die Zellzahl bei Aussaat bzw. Ernte und t die Kultivierungsdauer angibt.
Den Begriff "Ausbeute" beschreibt die prozentuale Widerfindung der jeweiligen Produktvarianten nach Auftrennung mittels Chromatographie an einer Matrix, z.B. einer Protein A Matrix.
Produktkonzentration von Proteinen, die von einer ausgewählten Nukleotidsequenz kodiert werden, können mit einem ELISA bestimmt werden aber auch durch andere Methoden, wie z.B. durch Protein A HPLC, Western Blot, Radioimmunassay, Immunpräzipitation, Nachweis der biologischen Aktivität des Proteins, Immunfärbung des Proteins mit nachfolgender FACS-Analyse oder Fluoreszenzmikroskopie, direkte Detektion eines fluoreszierenden Proteins mittel FACS-Analyse oder durch Spektrophotometrie.

### Gen von Interesse:

Das im erfindungsgemäßen Expressionsvektor enthaltene "Gen von Interesse" umfasst eine Nukleotidsequenz beliebiger Länge, die für ein Produkt von Interesse kodiert. Das Genprodukt oder auch "Produkt von Interesse" ist in der Regel ein Protein, Polypeptid, Peptid bzw. Fragment oder Derivat davon. Es kann aber auch RNA oder antisense RNA sein. Das Gen von Interesse kann in voller Länge, in verkürzter Form, als Fusionsgen oder markiertes Gen vorliegen. Es kann sich um genomische DNA oder vorzugsweise cDNA bzw. entsprechende Fragmente oder Fusionen handeln. Das Gen von Interesse kann die native Gensequenz darstellen, mutiert oder auf sonstige Weise modifiziert sein. Derartige Modifikationen schließen Kodon-Optimierungen zur Anpassung an eine bestimmte Wirtszelle und eine Humanisierung ein. Das Gen von Interesse kann z.B. für ein sekretiertes, zytoplasmatisches, kernlokalisiertes, membrangebundenes oder zelloberflächengebundenes Polypeptid kodieren.

Der Ausdruck "Nukleinsäure", "Nukleotidsequenz" oder "Nukleinsäuresequenz" bezeichnet ein Oligonukleotid, Nukleotide, Polynukleotide und deren Fragmente sowie DNA oder RNA genomischen oder synthetischen Ursprungs, die als Einzel- oder Doppelstrang vorliegen und den kodierenden oder den nicht-kodierenden Strang eines Gens repräsentieren kann. Zur Modifikation von Nukleinsäuresequenzen können Standardtechniken, wie z.B. ortsspezifische Mutagenese oder PCR-vermittelte Mutagenese, eingesetzt werden.

Unter "kodieren" versteht man die Eigenschaft oder Fähigkeit einer spezifischen Sequenz von Nukleotiden in einer Nukleinsäure, beispielsweise einem Gen in einem Chromosom oder einer mRNA, als Matrize für die Synthese von anderen Polymeren und Makromolekülen wie z.B. rRNA, tRNA, mRNA, anderen RNA-Molekülen, cDNA oder Polypeptiden in einem biologischen Prozess zu dienen. Demnach kodiert ein Gen für ein Protein, wenn durch Transkription und nachfolgende Translation der mRNA das gewünschte Protein in einer Zelle oder einem anderen biologischen System produziert wird. Sowohl der kodierende Strang, dessen Nukleotidsequenz identisch mit der mRNA-Sequenz ist und normalerweise auch in Sequenzdatenbanken, z.B. EMBL oder GenBank, angegeben wird, als auch der als Matrize für die Transkription dienende nichtkodierende Strang eines Gens oder cDNA kann dabei als kodierend für ein Produkt oder Protein bezeichnet werden. Eine Nukleinsäure, die für ein Protein kodiert, schließt auch Nukleinsäuren mit ein, die auf Grund des degenerierten genetischen Codes eine andere Nukleotidsequenzabfolge aufweisen, aber in der gleichen Aminosäuresequenz des Proteins resultieren. Nukleinsäuresequenzen, die für Proteine kodieren, können auch Introns enthalten.

Mit dem Ausdruck "cDNA" werden Desoxyribonukleinsäuren bezeichnet, die durch reverse Transkription und Synthese des zweiten DNA-Strangs aus einer von einem Gen produzierten mRNA oder anderen RNA hergestellt werden. Liegt die cDNA als doppelstränges DNA-Molekül vor, dann enthält sie sowohl einen kodierenden als auch einen nicht-kodierenden Strang.

### Protein/Produkt von Interesse:

Biopharmazeutisch bedeutsame Proteine/Polypeptide umfassen z.B. Antikörper bzw. Immunoglobuline, Enzyme, Cytokine, Lymphokine, Adhäsionsmoleküle, Rezeptoren sowie deren Derivate bzw. Fragmente, sind aber nicht auf diese beschränkt. Im Allgemeinen sind alle Polypeptide bedeutsam, die als Agonisten oder Antagonisten wirken und/oder therapeutische oder diagnostische Anwendung finden können. Andere Proteine von Interesse sind beispielsweise Proteine/Polypeptide, die zur Veränderung der Eigenschaften von Wirtszellen im Rahmen des sogenannten "Cell Engineering" verwendet werden, wie z.B. antiapoptotische Proteine, Chaperone, Stoffwechselenzyme, Glykosylierungsenzyme sowie deren Derivate bzw. Fragmente, sind aber nicht auf diese beschränkt.

Der Ausdruck "Polypeptide" wird für Aminosäuresequenzen oder Proteine verwendet und bezeichnet Polymere von Aminosäuren beliebiger Länge. Dieser Ausdruck schließt auch Proteine ein, die posttranslational durch Reaktionen wie beispielsweise Glykosylierung, Phosphorylierung, Acetylierung oder Proteinprozessierung modifiziert werden. Die Struktur des Polypeptids kann z.B. durch Substitutionen, Deletionen oder Insertion von Aminosäuren, Fusion mit anderen Proteinen, unter Beibehaltung seiner biologischen Aktivität modifiziert werden. Zudem können die Polypeptide multimerisieren und Homo- und Heteromere bilden.

"Immunoglobuline", oder auch "Antikörper" sind Proteine (Eiweiße) aus der Klasse der Globuline, die als Reaktion des Wirtsorganismus auf einen Fremdstoff (= Antigen) von ausdifferenzierten B-Lymphozyten (Plasmazellen) gebildet werden. Sie dienen der spezifischen Abwehr dieser Fremdstoffe. Es gibt verschiedene Klassen von Immunoglobulinen: IgA, IgD, IgE, IgG,IgM, IgY,IgW. Die Begriffe Immunoglobulin und Antikörper werden gleichbedeutend verwendet.

Beispiele für therapeutische Antikörper sind monoklonale, polyklonale, multispezifische und einzelkettige (*single chain*) Antikörper bzw. Immunoglobuline und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente. Die Antikörper können humanen oder nicht-humanen Ursprungs sein. Auch humanisierte und chimäre Antikörper kommen in Frage.

Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben.

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen ("*coiled coil structure*") wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).
Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VH/VL-Ketten. Die Diabodies können zusätzlich durch eingeführte Disulfidbrücken stabilisiert werden. Beispiele von Diabodies finden sich in der Literatur.
Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region.
Mit "Triabody" bezeichnet der Fachmann ein trivalentes homotrimeres scFv-Derivat. Die direkte Fusion von VH-VL ohne Verwendung einer Linkersequenz führt zur Ausbildung von Trimeren.
Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt.

### Herstellung erfindungsgemäßer Expressionsvektoren:

Die Herstellung des erfindungsgemäßen Expressionsvektors kann grundsätzlich nach herkömmlichen, dem Fachmann geläufigen Methoden erfolgen. Dort findet sich auch eine Beschreibung der funktionellen Komponenten eines Vektors, z.B. geeigneter Promotoren, Enhancer, Terminations- und Polyadenylierungssignale, Antibiotikaresistenzgene, Selektionsmarker, Replikationsstartpunkte und Spleisssignale. Zur Herstellung können herkömmliche Klonierungsvektoren verwendet werden, z.B. Plasmide, Bacteriophagen, Phagemide, Cosmide oder virale Vektoren wie Baculovirus, Retroviren, Adenoviren, Adeno-assoziierte Viren und Herpes Simplex-Virus, aber auch künstliche oder artifizielle oder Mini-Chromosomen. Die eukaryontischen Expressionsvektoren enthalten typischerweise auch prokaryontische Sequenzen wie z.B. Replikationsursprung und Antibiotikaresistenzgene, die die Vermehrung und Selektion des Vektors in Bakterien ermöglichen. Eine Vielzahl von eukaryontischen Expressionsvektoren, die multiple Klonierungsstellen zur Einführung einer Polynukleotidsequenz enthalten, sind bekannt und einige sind kommerziell bei verschiedenen Firmen wie Stratagene, La Jolla, CA, USA; Invitrogen, Carlsbad, CA, USA; Promega, Madison, WI, USA oder BD Biosciences Clontech, Palo Alto, CA, USA erhältlich.

Grundsätzlich kann die Expression der Gene innerhalb eines Expressionsvektors von einer oder mehreren Transkriptionseinheiten aus erfolgen. Als "Transkriptionseinheit" wird dabei eine Region definiert, die ein oder mehr zu transkribierende Gene enthält. Dabei sind die Gene innerhalb einer Transkriptionseinheit funktionell derart miteinander verknüpft, dass alle Gene innerhalb einer solchen Einheit unter der transkriptionellen Kontrolle desselben Promotors oder Promotors/Enhancers stehen. Als Resultat dieser transkriptionellen Verknüpfung von Genen kann mehr als ein Protein oder Produkt von einer Transkriptionseinheit aus transkribiert und somit exprimiert werden. Jede Transkriptionseinheit enthält dabei die regulatorischen Elemente, die für die Transkription und die Translation der in ihr enthaltenen Gensequenzen erforderlich sind. Jede Transkriptionseinheit kann dabei die gleichen oder verschiedene regulatorische Elemente enthalten. Für die funktionelle Verknüpfung der Gene innerhalb einer Transkriptionseinheit können IRES-Elemente oder Introns verwendet werden.

Der Expressionsvektor kann eine einzige Transkriptionseinheit zur Expression des Gens oder der Gene von Interesse und beispielsweise Selektionsmarkergene enthalten. Alternativ können diese Gene auch in zwei oder mehr Transkriptionseinheiten angeordnet sein. Dabei sind verschiedene Kombinationen der Gene innerhalb einer Transkriptionseinheit möglich. In einer weiteren Ausführungsform der vorliegenden Erfindung kann mehr als ein Expressionsvektor, bestehend aus ein, zwei oder mehr Transkriptionseinheiten, in eine Wirtszelle durch Co-Transfektion oder in aufeinanderfolgenden Transfektionen in beliebiger Reihenfolge eingeführt werden. Jede Kombination von regulatorischen Elementen und Genen auf jedem Vektor kann gewählt werden, so lange eine ausreichende Expression der Transkriptionseinheiten gewährleistet ist. Falls erforderlich können weitere regulatorische Elemente und Gene, wie z.B. zusätzliche Gene von Interesse oder Selektionsmarker, auf den Expressionsvektoren positioniert werden.

### Wirtszellen:

Zur Transfektion mit dem erfindungsgemäßen Expressionsvektor werden eukaryontische Wirtzellen verwendet, vorzugsweise Säugerzellen und insbesondere Nagerzellen wie z.B. Mäuse-, Ratten- und Hamster-Zelllinien. Die erfolgreiche Transfektion der entsprechenden Zellen mit einem erfindungsgemäßen Expressionsvektor resultiert in transformierten, genetisch modifizierten, rekombinanten oder transgenen Zellen, die ebenfalls Gegenstand der vorliegenden Erfindung sind.

Im Rahmen der Erfindung bevorzugte Wirtszellen sind Hamsterzellen wie z.B. BHK21, BHK TK⁻, CHO, CHO-K1, CHO-DUKX, CHO-DUKX B1 und CHO-DG44 Zellen oder Derivate/Abkömmlinge dieser Zelllinien. Besonders bevorzugt sind CHO-DG44, CHO-DUKX, CHO-K1 und BHK21 Zellen, insbesondere CHO-DG44 und CHO-DUKX Zellen. Ebenfalls geeignet sind Myelomzellen der Maus, vorzugsweise NS0 und Sp2/0 Zellen sowie Derivate/Abkömmlinge dieser Zelllinien. Aber auch Derivate und Abkömmlinge dieser Zellen, andere Säugerzellen, einschließlich aber nicht beschränkt auf Zelllinien von Mensch, Maus, Ratte, Affen, Nagetieren, oder eukaryontische Zellen, einschließlich aber nicht beschränkt auf Hefe-, Insekten- , Vogel- und Pflanzenzellen, können ebenfalls als Wirtszellen zur Produktion von biopharmazeutischen Proteinen verwendet werden.

Die Transfektion der eukaryontischen Wirtszellen mit einem Polynukleotid oder einem der erfindungsgemäßen Expressionsvektor, erfolgt nach üblichen Methoden. Geeignete Transfektionsmethoden sind z.B. die Liposomen-vermittelte Transfektion, Calciumphosphat-Copräzipitation, Elektroporation, Polykationen (z.B. DEAE-Dextran)-vermittelte Transfektion, Protoplastenfusion, Mikroinjektion und virale Infektionen. Erfindungsgemäß wird vorzugsweise eine stabile Transfektion durchgeführt, wobei die Konstrukte entweder in das Genom der Wirtszelle oder ein artifizielles Chromosom/Minichromosom integriert werden oder in stabiler Weise episomal in der Wirtszelle enthalten sind. Die Transfektionsmethode, die die optimale Transfektionsfrequenz und Expression des heterologen Gens in der jeweiligen Wirtszelle ermöglicht, ist dabei bevorzugt. Per Definition wird jede Sequenz oder jedes Gen, das in eine Wirtszelle eingebracht wird, in Bezug auf die Wirtszelle als "heterologe Sequenz" oder "heterologes Gen" bezeichnet. Selbst dann, wenn die einzubringende Sequenz oder das einzubringende Gen identisch zu einer endogenen Sequenz oder einem endogenen Gen der Wirtszelle ist. Beispielsweise ist ein Hamster Aktingen, das in eine Hamster-Wirtszelle eingebracht wird, per Definition ein heterologes Gen.

Bei der rekombinanten Herstellung heteromerer Proteine, wie z.B. monoklonaler Antikörper (mAk), kann die Transfektion geeigneter Wirtszellen prinzipiell auf zwei verschiedenen Wegen erfolgen. Derartige mAk sind aus mehreren Untereinheiten, den schweren und leichten Ketten, aufgebaut. Für diese Untereinheiten kodierende Gene können in unabhängigen oder in multicistronischen Transkriptionseinheiten auf einem einzigen Plasmid untergebracht werden, mit dem dann die Wirtszelle transfiziert wird. Dies soll die stöchiometrische Repräsentanz der Gene nach Integration in das Genom der Wirtszelle sichern. Allerdings muss hierbei im Falle unabhängiger Transkriptionseinheiten sichergestellt werden, dass die mRNAs, die für die verschiedenen Proteine kodieren, die gleiche Stabilität, Transkriptions- und Translationseffizienz aufweisen. Im zweiten Fall erfolgt die Expression der Gene innerhalb einer multicistronischen Transkriptionseinheit durch einen einzigen Promotor und es entsteht nur ein Transkript.
Durch Verwendung von IRES-Elementen wird eine recht effiziente interne Translationsinitiation der Gene in dem zweiten und den nachfolgenden Cistrons ermöglicht. Dennoch sind die Expressionsraten für diese Cistrons geringer als die des ersten Cistrons, dessen Translationsinitiation über einen sogenannten "cap"- abhängigen Prä-Initiationskomplex wesentlich effizienter ist als die IRESabhängige Translationsinitiation. Um eine tatsächlich äquimolare Expression der Cistrons zu erreichen, können beispielsweise noch zusätzliche intercistronische Elemente eingeführt werden, die im Zusammenwirken mit den IRES-Elementen für einheitliche Expressionsraten sorgen.
Eine andere und erfindungsgemäß bevorzugte Möglichkeit der simultanen Herstellung mehrerer heterologer Proteine ist die Co-Transfektion, bei der die Gene getrennt in verschiedene Expressionsvektoren integriert werden. Dies hat den Vorteil, dass bestimmte Verhältnisse der Gene und Genprodukte zueinander eingestellt werden können, wodurch Unterschiede in der mRNA-Stabilität sowie in der Transkriptions- und Translationseffizienz ausgeglichen werden können. Außerdem sind die Expressionsvektoren wegen ihrer geringeren Größe stabiler und sowohl bei der Klonierung als auch bei der Transfektion einfacher handhabbar.

In einer besonderen Ausführungsform der Erfindung werden daher die Wirtszellen zusätzlich mit einem oder mehreren Vektoren mit Genen, die für ein oder mehrere andere Proteine von Interesse kodieren, transfiziert, bevorzugt co-transfiziert. Der oder die zur Co-Transfektion verwendeten weiteren Vektoren kodieren z.B. für das oder die anderen Proteine von Interesse unter der Kontrolle des gleichen Promotors, bevorzugt unter der Kontrolle der gleichen Promotor/Enhancer-Kombination sowie für zumindest einen Selektionsmarker, z.B. die Dihydrofolat-Reduktase.

In einer weiteren besonderen Ausführungsform der Erfindung werden die Wirtszellen mit zumindest zwei eukaryontischen Expressionsvektoren co-transfiziert, wobei zumindest der eine der beiden Vektoren zumindest ein Gen, das zumindest Protein von Interesse kodiert, enthält und der andere Vektor ein oder mehrere erfindungsgemäße Nukleinsäuren in beliebiger Kombination, Position und Orientierung enthält, und optional auch für zumindest ein Gen (von Interesse kodiert, und diese erfindungsgemäßen Nukleinsäuren ihre transkriptions- bzw. expressionssteigernde Wirkung auf die Gene von Interesse, die auf dem anderen co-transfizierten Vektor lokalisiert sind, via Co-Integration mit dem anderen Vektor vermitteln.

Erfindungsgemäß werden die Wirtszellen vorzugsweise unter serumfreien Bedingungen etabliert, adaptiert und kultiviert, gegebenenfalls in Medien, die frei von tierischen Proteinen /Peptiden sind. Beispiele für im Handel erhältliche Medien sind Ham's F12 (Sigma, Deisenhofen, DE), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA, USA), CHO-S-SFMII (Invitrogen), serumfreies CHO-Medium (Sigma) und proteinfreies CHO-Medium (Sigma). Jedes dieser Medien kann gegebenenfalls mit verschiedenen Verbindungen ergänzt werden, z.B. Hormonen und/oder anderen Wachstumsfaktoren (z.B. Insulin, Transferrin, epidermalem Wachstumsfaktor, Insulin-ähnlichem Wachstumsfaktor), Salzen (z.B. Natriumchlorid, Calcium, Magnesium, Phosphat), Puffern (z.B. HEPES), Nukleosiden (z.B. Adenosin, Thymidin), Glutamin, Glukose oder anderen äquivalenten Nährstoffen, Antibiotika und/oder Spurenelementen. Obwohl erfindungsgemäß serumfreie Medien bevorzugt sind, können zur Züchtung der Wirtszellen auch Medien verwendet werden, die mit einer geeigneten Menge an Serum versetzt wurden. Zur Selektion von genetisch modifizierten Zellen, die ein oder mehrere Selektionsmarkergene exprimieren, wird dem Medium ein oder mehrere geeignete Selektionsmittel zugefügt.

Als "Selektionsmittel" wird eine Substanz bezeichnet, die das Wachstum oder das Überleben von Wirtszellen mit einer Defizienz für das jeweilige Selektionsmarkergen beeinträchtigt. Im Rahmen der vorliegenden Erfindung wird vorzugsweise Geneticin (G418) als Mediumzusatz zur Selektion heterologer Wirtszellen, die ein Wildtyp- oder vorzugsweise ein modifiziertes Neomycin-Phosphotransferase-Gen tragen, verwendet. Sollten die Wirtzellen mit mehreren Expressionsvektoren transfiziert werden, z.B. wenn separat mehrere Gene von Interesse in die Wirtszelle eingebracht werden sollen, so verfügen diese meist über verschiedene Selektionsmarkergene.

Ein "Selektionsmarkergen" ist ein Gen, das die spezifische Selektion von Zellen, die dieses Gen erhalten, durch Zugabe eines entsprechenden Selektionsmittels in das Kultivierungsmedium ermöglicht. Zur Verdeutlichung, ein Antibiotika-Resistenzgen kann als positiver Selektionsmarker verwendet werden. Nur Zellen, die mit diesem Gen transformiert wurden, können in der Gegenwart des entsprechenden Antibiotikums wachsen und somit selektioniert werden. Nichttransfomierte Zellen hingegen können unter diesen Selektionsbedingungen nicht wachsen oder überleben. Es gibt positive, negative und bifunktionale Selektionsmarker. Positive Selektionsmarker ermöglichen die Selektion und damit Anreicherung von transformierten Zellen durch Vermittlung einer Resistenz gegenüber dem Selektionsmittel oder durch Kompensierung eines metabolischen oder katabolischen Defekts der Wirtszelle. Im Gegensatz dazu können durch negative Selektionsmarker Zellen, die das Gen für den Selektionsmarker erhalten haben, selektiv eliminiert werden. Ein Beispiel hierfür ist das Thymidinkinasegen des Herpes Simplex Virus, dessen Expression in Zellen bei gleichzeitiger Gabe von Acyclovir oder Gancyclovir zu deren Elimination führt. Die in dieser Erfindung verwendeten Selektionsmarker, einschließlich der amplifzierbaren Selektionsmarker, schließt gentechnologisch veränderte Mutanten und Varianten, Fragmente, funktionelle Äquivalente, Derivate, Homologe und Fusionen mit anderen Proteinen oder Peptiden ein, solange der Selektionsmarker seine selektiven Eigenschaften beibehält. Solche Derivate weisen eine beträchtliche Homologie in der Aminosäuresequenz in den Bereichen oder Domänen auf, denen die selektive Eigenschaft zugeschrieben wird. In der Literatur sind eine Vielzahl von Selektionsmarkergenen, einschließlich bifunktionaler (positiv/negativ) Marker, beschrieben. Beispiele für Selektionsmarker, die für gewöhnlich in eukaryontischen Zellen verwendet werden, beinhalten die Gene für Aminoglykosid-Phosphotransferase (APH), Hygromycin-Phosphotransferase (HYG), Dihydrofolat-Reduktase (DHFR), Thymidinkinase (TK), Glutamin-Synthetase, Asparagin-Synthetase und Gene, die Resistenz gegenüber Neomycin (G418), Puromycin, Histidinol D, Bleomycin, Phleomycin und Zeocin vermitteln.

### Amplifizierbares Selektionsmarkergen:

Ferner können die erfindungsgemäßen Zellen optional auch einem oder mehreren Genamplifikationsschritten unterzogen werden, in dem sie in Gegenwart eines Selektionsmittels kultiviert werden, das zu einer Amplifikation eines amplifzierbaren Selektionsmarkergens führt.
Voraussetzung ist, dass die Wirtszellen zusätzlich mit einem Gen transfiziert werden, das für einen amplifizierbaren Selektionsmarker kodiert. Denkbar ist, dass das Gen, welches für einen amplifizierbaren Selektionsmarker kodiert, auf einem der erfindungsgemäßen Expressionsvektoren vorhanden ist oder aber mit Hilfe eines weiteren Vektors in die Wirtszelle eingebracht wird.

Das amplifizierbare Selektionsmarkergen kodiert gewöhnlich für ein Enzym, das für das Wachstum von eukaryontischen Zellen unter bestimmten Kultivierungsbedingungen erforderlich ist. Beispielsweise kann das amplifizierbare Selektionsmarkergen für Dihydrofolat-Reduktase (DHFR) kodieren. In diesem Fall wird das Gen amplifiziert, wenn man eine damit transfizierte Wirtszelle in Gegenwart des Selektionsmittels Methotrexat (MTX) kultiviert. Der DHFR-Marker ist bei der Verwendung von DHFR-negativen Grundzellen wie CHO-DG44 oder CHO-DUKX besonders gut zur Selektion und nachfolgenden Amplifikation geeignet, da diese Zellen kein endogenes DHFR exprimieren und somit nicht in Purin-freiem Medium wachsen. Deshalb kann hier das DHFR-Gen als dominanter Selektionsmarker eingesetzt werden und die transformierten Zellen werden in Hypoxanthin/Thymidin-freiem Medium selektioniert.
Weitere erfindungsgemäß verwendbare amplifizierbare Selektionsmarkergene sind beispielsweise Glutamin-Synthetase , Methallothionein, Adenosin-Deaminase, AMP-Deaminase, UMP-Synthase, Xanthin-Guanin-Phosphoribosyltransferase und Thymdilat-Synthetase.

### Genexpression und Selektion hoch produzierender Wirtszellen:

Der Ausdruck "Genexpression" oder "Expression" bezieht sich auf die Transkription und/oder Translation einer heterologen Gensequenz in einer Wirtszelle. Die Expressionsrate kann hierbei allgemein bestimmt werden, entweder auf Basis der Menge der entsprechenden mRNA, die in der Wirtszelle vorhanden ist, oder auf Basis der produzierten Menge an Genprodukt, das von dem Gen von Interesse kodiert wird. Die Menge der durch Transkription einer ausgewählten Nukleotidsequenz erzeugten mRNA kann beispielsweise durch Northern Blot Hybridisierung, Ribonuklease-RNA-Protektion, *in situ* Hybridisierung von zellulärer RNA oder durch PCR-Methoden (z.B. quantitativer PCR) bestimmt werden. Proteine, die von einer ausgewählten Nukleotidsequenz kodiert werden, können ebenfalls durch verschiedene Methoden, wie z.B. durch ELISA, Protein A HPLC, Western Blot, Radioimmunassay, Immunpräzipitation, Nachweis der biologischen Aktivität des Proteins, Immunfärbung des Proteins mit nachfolgender FACS-Analyse oder Fluoreszenzmikroskopie, direkte Detektion eines fluoreszierenden Proteins mittel FACS-Analyse oder durch Fluoreszenzmikroskopie bestimmt werden.

Mit "Titer- oder Produktivitätssteigerung" wird die Steigerung der Expression, Synthese oder Sekretion einer in eine Wirtszelle eingebrachten heterologen Sequenz, beispielsweise eines für ein therapeutisches Protein kodierenden Gens, im Vergleich zu einer geeigneten Kontrolle, beispielsweise Mutanten-Protein versus Wildtyp-Protein, bezeichnet. Eine Titer- oder Produktivitätssteigerung liegt vor, wenn eine erfindungsgemäße Zelle nach einem hier beschriebenen erfindungsgemäßen Verfahren kultiviert wird, und wenn diese Zelle mindestens eine Steigerung der spezifischen Produktivität oder des Titers um 10 % aufweist. Eine Titer- oder Produktivitätssteigerung liegt auch dann vor, wenn eine erfindungsgemäße Zelle nach einem hier beschriebenen erfindungsgemäßen Verfahren kultiviert wird, und wenn diese Zelle mindestens eine Steigerung der spezifischen Produktivität oder des Titers um 20 % bzw. mindestens 50% bzw. mindestens 75% aufweist. Eine Titer- oder Produktivitätssteigerung liegt insbesondere auch dann vor, wenn eine erfindungsgemäße Zelle nach einem hier beschriebenen erfindungsgemäßen Verfahren kultiviert wird, und wenn diese Zelle mindestens eine Steigerung der spezifischen Produktivität oder des Titers um 10-500 % aufweist, bevorzugt 20-300%, besonders bevorzugt 50-200%.
Eine Titer- oder Produktivitätssteigerung kann sowohl durch Verwendung einer der erfindungsgemäßen Expressionsvektoren als auch durch Anwendung eines der erfindungsgemäßen Verfahren erreicht werden.

Die entsprechenden Verfahren können mit einer durch FACS gestützten Selektion von rekombinanten Wirtszellen, die als weiteren Selektionsmarker beispielsweise ein (oder mehrere) Fluoreszenzprotein(e) (z.B. GFP) oder einen Zelloberflächenmarker enthalten, kombiniert werden. Andere Methoden zur Erzielung einer verstärkten Expression, wobei auch eine Kombination verschiedener Methoden möglich ist, beruhen beispielsweise auf der Verwendung von *cis*-aktiven Elementen zur Manipulation der Chromatinstruktur (z.B. LCR, UCOE, EASE, Isolatoren, S/MARs, STAR-Elemente), Verwendung von (artifiziellen) Transkriptionsfaktoren, Behandlung der Zellen mit natürlichen oder synthetischen Agenzien zur Hochregulation endogener oder heterologer Genexpression, Verbesserung der Stabilität (Halbwertszeit) der mRNA oder des Proteins, Verbesserung der mRNA-Translationsinitiation, Erhöhung der Gendosis durch Verwendung von episomalen Plasmiden (basierend auf der Verwendung von viralen Sequenzen als Replikationsursprung, z.B. von SV40, Polyoma, Adenovirus, EBV oder BPV), Einsatz von amplifikationsfördernden Sequenzen oder auf DNA-Konkatemeren basierenden *in vitro* Amplifikationssystemen.

Weiterhin erfindungsgemäß ist ein Verfahren, bei dem Produktionszellen vermehrt und zur Herstellung des kodierenden Genprodukt von Interesse verwendet werden. Hierzu werden die selektionierten hoch produzierenden Zellen vorzugsweise in einem serumfreien Kulturmedium und vorzugsweise in Suspensionskultur unter Bedingungen gezüchtet, die eine Expression des Gens von Interesse erlauben. Das Protein/Produkt von Interesse wird dabei vorzugsweise als sekretiertes Genprodukt aus dem Zellkulturmedium gewonnen. Bei Expression des Proteins ohne Sekretionssignal kann das Genprodukt aber auch aus Zelllysaten isoliert werden. Um ein reines, homogenes Produkt zu erhalten, das im Wesentlichen frei ist von anderen rekombinanten Proteinen und Wirtszellproteinen, werden übliche Reinigungsschritte durchgeführt. Hierzu entfernt man häufig zunächst Zellen und Zelltrümmer aus dem Kulturmedium oder Lysat. Das gewünschte Genprodukt kann dann von kontaminierenden löslichen Proteinen, Polypeptiden und Nukleinsäuren befreit werden, z.B. durch Fraktionierung an Immunaffinitäts- und Ionenaustauschsäulen, Ethanolfällung, Umkehrphasen-HPLC oder Chromatographie an Sephadex, Silica oder Kationenaustauscherharzen wie DEAE. Methoden, die zur Reinigung eines von rekombinanten Wirtszellen exprimierten heterologen Proteins führen, sind dem Fachmann bekannt und sind in der Literatur beschrieben.

### ERFINDUNGSGEMÄSSE AUSFÜHRUNGSFORMEN

Die vorliegende Erfindung betrifft ein Verfahren zur Steigerung des Titers eines Proteins von Interesse einer Zelle dadurch gekennzeichnet, dass
a. in einer Nukleinsäuresequenz, die für das Protein von Interesse kodiert, mindestens das Kodon deletiert wird, das für die C-terminale Aminosäure kodiert,
b. die Zelle mit einem Vektor transfiziert wird, welcher die veränderte Nukleinsäure aus a) enthält und
c. die Zelle unter Bedingungen kultiviert wird, die eine Produktion des Proteins von Interesse erlaubt.

Insbesondere betrifft die vorliegende Erfindung ein Verfahren zur Steigerung des Titers eines Antikörpers einer Zelle dadurch gekennzeichnet, dass in einer Nukleinsäuresequenz, die für die schwere Kette des Antikörpers kodiert, mindestens das Kodon deletiert wird, das für die C-terminale Aminosäure Lysin kodiert, die Zelle mit einem Vektor transfiziert wird, welcher die veränderte Nukleinsäure enthält, und die Zelle unter Bedingungen kultiviert wird, die eine Produktion des Antikörpers von Interesse erlaubt.

Die vorliegende Erfindung betrifft bevorzugt ein Verfahren zur Steigerung der spezifischen Produktivität eines Proteins von Interesse einer Zelle dadurch gekennzeichnet, dass in einer Nukleinsäuresequenz, die für das Protein von Interesse kodiert, mindestens das Kodon deletiert wird, das für die C-terminale Aminosäure kodiert, die Zelle mit einem Vektor transfiziert wird, welcher die veränderte Nukleinsäure enthält, und die Zelle unter Bedingungen kultiviert wird, die eine Produktion des Proteins von Interesse erlaubt.
In einer besonders bevorzugten Ausführungsform betrifft die vorliegende Erfindung ein Verfahren zur Steigerung der spezifischen Produktivität eines Antikörpers einer Zelle dadurch gekennzeichnet, dass in einer Nukleinsäuresequenz, die für die schwere Kette des Antikörpers kodiert, mindestens das Kodon deletiert wird, das für die C-terminale Aminosäure Lysin kodiert, die Zelle mit einem ersten Vektor transfiziert wird, welcher die veränderte Nukleinsäure enthält, die Zelle mit einem zweiten Vektor co-transfiziert wird, welcher die leichte Kette eines Antikörpers enthält, und die Zelle unter Bedingungen kultiviert wird, die eine Produktion des Antikörpers erlaubt.

In einem weiteren bevorzugten Verfahren werden die modifizierte schwere Kette und die leichte Kette, bzw. die Untereinheiten eines heteromeren Proteins, in aufeinander folgenden Transfektionen in beliebiger Reihenfolge eingeführt.
In einer weiteren bevorzugten Ausführungsform betrifft die vorliegende Erfindung ein Verfahren zur Steigerung der spezifischen Produktivität eines Antikörpers oder eines beliebigen heteromeren Proteins von Interesse einer Zelle dadurch gekennzeichnet, dass in einer Nukleinsäuresequenz, die für die schwere Kette des Antikörpers kodiert, mindestens das Kodon deletiert wird, das für die C-terminale Aminosäure Lysin kodiert, die Zelle mit einem Vektor transfiziert wird, welcher sowohl die veränderte Nukleinsäure für die schwere Kette eines Antikörpers enthält als auch die leichten Kette eines Antikörpers, und die Zelle unter Bedingungen kultiviert wird, die eine Produktion des Antikörpers erlaubt. In einer bevorzugten Ausführungsform des Verfahrens ist der Vektor, mit welchem die Zelle transfiziert wird, ein bi- oder multicistronischer Vektor. In einer weiteren bevorzugten Ausführungsform des Verfahrens ist der Vektor, mit welchem die Zelle transfiziert wird, ein Vektor, welcher die schwere und leichte Antikörperkette als separate Transkriptionseinheiten enthält,

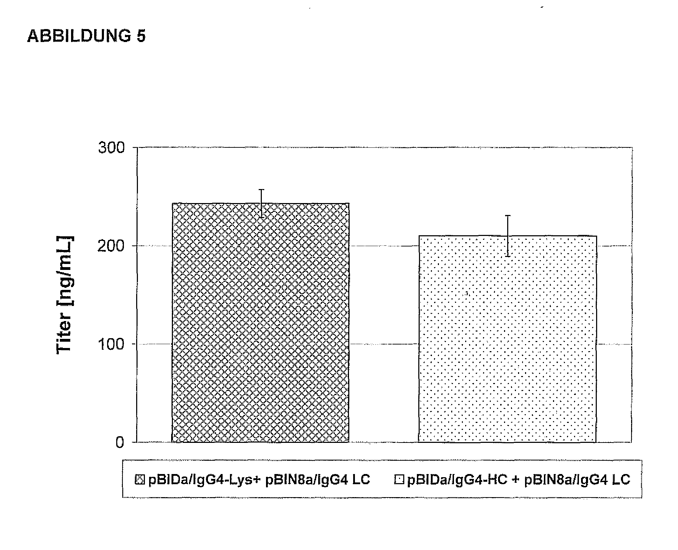

Es kann überraschender Weise gezeigt werden, dass z.B. ein IgG1-Molekül trotz der Deletion des C-terminalen Lysins in CHO-Zellen exprimiert und sezerniert wird und die Produktmengen vergleichbar mit der von IgG1 Wildtyp transfizierten Zellen ist (Abb. 2). Weiterhin zeigt sich überraschender Weise, dass Zellen, die die Lysin-Deletionsvariante des IgG1 exprimieren, durchschnittlich sogar 27% höhere Titer bzw. 32% höhere spezifische Produktivitäten erzielen als Zellen, die den IgG1-Wildtyp exprimieren (Abb. 3). Dieser Produktionsvorteil der Lysin-Deletionsvariante ist auch dann noch gegeben, wenn in diesen Zellpools durch Zugabe von 100 nM MTX eine DHFR-basierte Genamplifikation induziert wird. Die Titer und spezifischen Produktivitäten sind durchschnittlich um 86% bzw. 120% höher (Abb. 4).
Ähnliches kann überraschender Weise auch für ein IgG4-Molekül gezeigt werden. Das IgG4-Molekül wird trotz der Deletion des C-terminalen Lysins in CHO-Zellen sogar etwas besser exprimiert und sezerniert als der IgG4 Wildtyp (Abb. 5). Überraschender Weise zeigt sich, dass Zellen, die die Lysin-Deletionsvariante des IgG4s exprimieren, durchschnittlich sogar 63 % höhere Titer bzw. 70 % höhere spezifische Produktivitäten erzielen als Zellen, die den IgG4-Wildtyp exprimieren (Abb. 6). Dieser Produktionsvorteil der Lysin-Deletionsvariante ist auch bei dem nachfolgenden Amplifikationsschritt mit 400 nM MTX gegeben. Hier werden im Durchschnitt 53% höhere Titer und 66% höhere spezifischen Produktivitäten erzielt (Abb. 7).

In einer speziellen Ausführungsform des erfindungsgemäßen Verfahrens ist der Titer und /oder die spezifische Produktivität um 10-500 %, bevorzugt 20-300%, besonders bevorzugt 50-200% gesteigert in Bezug auf den Vergleichswert des Proteins ohne die Deletion der C-terminalen Aminosäure. In einer weiteren speziellen Ausführungsform des erfindungsgemäßen Verfahrens ist der Titer und /oder die spezifische Produktivität um mindestens 10 %, bevorzugt um mindestens 20 %, weiterhin bevorzugt um mindestens 50 %, und besonders bevorzugt um mindestens 75 % gesteigert in Bezug auf den Vergleichswert des Proteins ohne die Deletion der C-terminalen Aminosäure.
In einer weiteren speziellen Ausführungsform des erfindungsgemäßen Verfahrens beträgt die spezifische Produktivität mindestens 5 pg/Zelle/Tag.

Die vorliegende Erfindung betrifft weiterhin ein Verfahren zur Erzeugung eines Expressionsvektors zur gesteigerten Produktion eines Proteins von Interesse dadurch gekennzeichnet, dass in der Nukleinsäuresequenz, die für das Protein von Interesse kodiert, mindestens das Kodon deletiert wird, das für die C-terminale Aminosäure kodiert, und die so modifizierte Nukleinsäuresequenz in einen Expressionsvektor eingeführt wird.

Die vorliegende Erfindung betrifft weiterhin ein Verfahren zur Erzeugung einer Zelle mit gesteigertem Titer und / oder gesteigerter spezifischer Produktivität eines Proteins von Interesse dadurch gekennzeichnet, dass eine Zelle gemäß einem erfindungsgemäßen Verfahren behandelt wird und anschließend eine Einzelzellklonierung vorgenommen wird, z.B. über Verdünnungsklonierung oder FACS basierte Einzellzellablage.

Die vorliegende Erfindung betrifft weiterhin ein Verfahren zur Herstellung eines Proteins von Interesse in einer Zelle dadurch gekennzeichnet, dass eine Gruppe von Zellen gemäß einem erfindungsgemäßen Verfahren behandelt wird, diese Zellen in Anwesenheit zumindestens eines Selektionsdrucks selektioniert werden, optional eine Einzelzellklonierung vorgenommen wird und das Protein von Interesse aus den Zellen oder dem Kulturüberstand gewonnen wird.

Eine spezielle Ausführungsform des erfindungsgemäßen Verfahrens zur Herstellung mindestens eines Proteins von Interesse ist dadurch gekennzeichnet, dass die zur Herstellung verwendeten Zellen nach dem Selektionsschritt mittels Selektionsmittel zusätzlich einem Genamplifikationsschritt unterzogen werden.

Eine spezifische Ausführungsform aller genannten erfindungsgemäßen Verfahren ist dadurch gekennzeichnet, dass die C-terminale Aminosäure Lysin (Lys) oder Arginin (Arg) ist, bevorzugt Lys.

Eine weitere spezifische Ausführungsform aller genannten erfindungsgemäßen Verfahren ist dadurch gekennzeichnet, dass das Protein von Interesse ein Antikörper, ein Fc-Fusionsprotein, EPO oder tPA ist.

Eine bevorzugte Ausführungsform aller genannten erfindungsgemäßen Verfahren ist dadurch gekennzeichnet, dass das Protein von Interesse eine schwere Kette eines Antikörpers ist und die C-terminale Aminosäure Lysin (Lys).

Eine spezielle Ausführungsform aller genannten erfindungsgemäßen Verfahren ist dadurch gekennzeichnet, dass die schwere Kette des Antikörpers vom Typ IgG1, IgG2, IgG3 oder IgG4 ist, bevorzugt vom Typ IgG1, IgG4 oder IgG2.

Eine spezifische Ausführungsform aller genannten erfindungsgemäßen Verfahren ist dadurch gekennzeichnet, dass das Protein von Interesse ein monoklonaler, polyklonaler, Säugetier-, Maus-, chimärer, humanisierter, Primaten- oder ein humaner Antikörper ist oder ein Antikörperfragment oder -derivat einer schweren Kette eines Immunoglobulin Antikörpers ist oder eines Fab, F(ab')2, Fc, Fc-Fc Fusionsproteins, Fv, single chain Fv, single domain Fv, tetravalenten single chain Fv, Disulfid-verknüpften Fv, Domänen-deletierten Antikörpers, eines Minibodys, Diabodys, oder eines Fusionspolypeptides eines der genannten Fragmente mit einem anderen Peptid oder Polypeptid ist oder ein Fc-Peptid Fusionsprotein, ein Fc-Toxin Fusionsprotein oder ein Scaffold-Protein ist.

Eine weitere spezifische Ausführungsform aller genannten erfindungsgemäßen Verfahren ist dadurch gekennzeichnet, dass die Zelle in Suspensionskultur kultiviert wird. Eine spezielle Ausführungsform aller genannten erfindungsgemäßen Verfahren ist dadurch gekennzeichnet, dass die Zelle serumfrei kultiviert wird. Eine weitere spezielle Ausführungsform aller genannten erfindungsgemäßen Verfahren ist dadurch gekennzeichnet, dass die Zelle in chemisch definiertem Medium kultiviert wird. Eine bevorzugte Ausführungsform aller genannten erfindungsgemäßen Verfahren ist dadurch gekennzeichnet, dass die Zelle in proteinfreiem Medium kultiviert wird.
Eine bevorzugte Ausführungsform aller genannten erfindungsgemäßen Verfahren ist dadurch gekennzeichnet, dass die Zelle eine eukaryotische Zelle ist, z.B. aus Hefe, Pflanzen, Würmern, Insekten, Vögeln, Fischen, Reptilien oder Säugern. Eine weitere bevorzugte Ausführungsform aller genannten erfindungsgemäßen Verfahren ist dadurch gekennzeichnet, dass die Zelle eine Säugerzelle ist. Eine besonders bevorzugte Ausführungsform aller genannten erfindungsgemäßen Verfahren ist dadurch gekennzeichnet, dass die Zelle eine CHO Zelle ist. Eine weitere spezielle Ausführungsform der genannten erfindungsgemäßen Verfahren ist dadurch gekennzeichnet, dass die CHO Zelle ausgewählt ist aus der Gruppe:
CHO wild type, CHO K1, CHO DG44, CHO DUKX-B11 und CHO Pro-5. Besonders bevorzugt ist eine CHO DG44 Zelle.

Die Erfindung betrifft weiterhin einen Expressionsvektor mit gesteigerter Expression eines Gens von Interesse generierbar nach einem der genannten erfindungsgemäßen Verfahren.

Die Erfindung betrifft weiterhin eine Zelle generierbar nach einem der genannten erfindungsgemäßen Verfahren.

Die Erfindung betrifft weiterhin ein Verfahren zur Produktion und Reinigung eines Proteins von Interesse dadurch gekennzeichnet, dass mindestens eine C-terminale Aminosäure des entsprechenden Gens von Interesse deletiert ist und das erhaltene Protein von Interesse eine verringerte Heterogenität aufweist im Vergleich zum Wildtyp-Protein ohne Deletion.

Eine spezielle Ausführungsform des erfindungsgemäßen Verfahrens ist dadurch gekennzeichnet, dass die C-terminale Aminosäure Lysin (Lys) oder Arginin (Arg) ist, bevorzugt Lys.

Eine weitere spezielle Ausführungsform des erfindungsgemäßen Verfahrens ist dadurch gekennzeichnet, dass das Protein von Interesse ein Antikörper, ein Fc-Fusionsprotein, EPO oder tPA ist.

Eine bevorzugte Ausführungsform des erfindungsgemäßen Verfahrens ist dadurch gekennzeichnet, dass das Protein von Interesse eine schwere Kette eines Antikörpers ist und die C-terminale Aminosäure Lysin (Lys).

Eine weitere bevorzugte Ausführungsform des erfindungsgemäßen Verfahrens ist dadurch gekennzeichnet, dass die schwere Kette des Antikörpers vom Typ IgG1, IgG2, IgG3 oder IgG4 ist, bevorzugt vom Typ IgG1, IgG2 oder IgG4.
Eine spezielle Ausführungsform des erfindungsgemäßen Verfahrens ist dadurch gekennzeichnet, dass für die Produktion Zellen in Suspensionskultur kultiviert werden. Eine weitere spezielle Ausführungsform des erfindungsgemäßen Verfahrens ist dadurch gekennzeichnet, dass für die Produktion Zellen serumfrei kultiviert werden. Eine weitere spezielle Ausführungsform aller genannten erfindungsgemäßen Verfahren ist dadurch gekennzeichnet, dass die Zelle in chemisch definiertem Medium kultiviert wird. Eine bevorzugte Ausführungsform aller genannten erfindungsgemäßen Verfahren ist dadurch gekennzeichnet, dass die Zelle in proteinfreiem Medium kultiviert wird.
Eine bevorzugte Ausführungsform des erfindungsgemäßen Verfahrens ist dadurch gekennzeichnet, dass die Zellen Säugerzellen sind.
Eine weitere bevorzugte Ausführungsform des erfindungsgemäßen Verfahrens ist dadurch gekennzeichnet, dass die Zellen CHO Zellen sind, bevorzugt CHO DG44 Zellen.
Eine besonders bevorzugte Ausführungsform des erfindungsgemäßen Verfahrens ist dadurch gekennzeichnet, dass bei der Reinigung des Proteins von Interesse eine niedrigere Salzkonzentration zum Einsatz kommt im Vergleich zur Reinigung eines Wildtyp-Proteins ohne Deletion.

Die Erfindung betrifft weiterhin ein Verfahren zur Herstellung eines Antikörpers dadurch gekennzeichnet, dass in einer Nukleinsäure, die für die schwere Kette eines Antikörpers kodiert, mindestens das Kodon deletiert wird, das für die C-terminale Aminosäure Lysin kodiert, die Zelle mit einem Vektor transfiziert wird, welcher die so veränderte Nukleinsäure enthält, und die Zelle unter Bedingungen kultiviert wird, die eine Expression des Antikörpers erlaubt.

Im folgenden wird die Erfindung anhand nicht-beschränkender Ausführungsbeispiele näher erläutert.

### BEISPIELE

**ABKÜRZUNGEN**

| | |
|---|---|
| AP: | alkalische Phosphatase |
| Asp (=D): | Asparaginsäure |
| bp: | Basenpaar |
| CHO: | Chinese Hamster Ovary |
| CpB: | Carboxypeptidase B |
| DHFR: | Dihydrofolat-Reduktase |
| ELISA: | enzyme-linked immunosorbant assay |
| HT: | Hypoxanthin/Thymidin |
| IgG: | Immunoglobulin G |
| Ile (=I): | Isoleucin |
| kb: | Kilobase |
| Lys: | Lysin |
| mAk: | monoklonaler Antikörper |
| MTX: | Methotrexat |
| MW: | Mittelwert |
| NPT: | Neomycin-Phosphotransferase |
| PCR: | polymerase chain reaction |
| Phe (=F): | Phenylalanin |
| SEAP: | secreted alkaline phosphatase |
| WT: | Wildtyp |

### METHODEN

### Zellkultur und Transfektion

Die Zellen CHO-DG44/dhfr^{-/-} werden permanent als Suspensionszellen in serumfreiem und mit Hypoxanthin und Thymidin (HT) supplementiertem CHO-S-SFMII Medium (Invitrogen GmbH, Karlsruhe, DE) in Zellkulturflaschen bei 37°C in feuchter Atmosphäre und 5% CO₂ kultiviert. Die Zellzahlen sowie die Viabilität werden mit einem Cedex (Innovatis) bestimmt und die Zellen dann in einer Konzentration von 1 - 3 x10⁵/mL eingesät und alle 2 - 3 Tage passagiert.
Zur Transfektion von CHO-DG44 wird Lipofectamine Plus Reagenz (Invitrogen) eingesetzt. Pro Transfektionsansatz wird dabei insgesamt 1,0 - 1,1 µg Plasmid-DNA, 4 µL Lipofectamine und 6 µL Plus-Reagenz nach den Angaben des Herstellers gemischt und in einem Volumen von 200 µL zu 6x10⁵ Zellen in 0,8 ml HT-supplementiertem CHO-S-SFMII Medium gegeben. Nach dreistündiger Inkubation bei 37°C in einem Zellinkubator erfolgt eine Zugabe von 2 mL HT-supplementiertem CHO-S-SFMII Medium. Nach einer Kultivierungszeit von 48 Stunden werden die Transfektionsansätze entweder geerntet (transiente Transfektion) oder einer Selektion unterworfen. Da der eine Expressionsvektor einen DHFR- und der andere einen NPT-Selektionsmarker enthält, werden die co-transfizierten Zellen zur DHFR- und NPT-basierten Selektion 2 Tage nach Transfektion in CHO-S-SFMII Medium ohne Hypoxanthin- und Thymidinzusatz transferiert und dem Medium außerdem noch G418 (Invitrogen) in einer Konzentration von 400 µg/mL zugesetzt.
Eine DHFR-basierte Genamplifikation der integrierten heterologen Gene wird durch Zugabe des Selektionsagens MTX (Sigma, Deisenhofen, DE) in einer Konzentration von 5 - 2000 nM zu einem HT-freien CHO-S-SFMII Medium erreicht.

### Expressionsvektoren

Zur Expressionsanalyse werden eukaryontische Expressionsvektoren eingesetzt, die auf dem pAD-CMV Vektor basieren und die Expression eines heterologen Gens über die Kombination CMV Enhancer/Hamster Ubiquitin/S27a Promotor (WO 97/15664) oder CMV Enhancer/CMV Promotor vermitteln. Während der Basisvektor pBID das dhfr-Minigen enthält, das als amplifzierbarer Selektionsmarker dient, ist im Vektor pBIN das dhfr-Minigen durch ein NPT-Gen ersetzt. Hierzu wurde der NPT-Selektionsmarker, inklusive SV40 early Promotor und TK-Polyadenylierungssignal, aus dem kommerziellen Plasmid pBK-CMV (Stratagene, La Jolla, CA, USA) als 1640 bp Bsu361-Fragment isoliert. Nach einer Auffüllreaktion der Fragmentenden durch Klenow-DNA-Polymerase wurde das Fragment mit dem 3750 bp Bsu361/Stul-Fragment des Vektors pBID, das ebenfalls mit Klenow-DNA-Polymerase behandelt wurde, ligiert. In beiden Vektoren wird die Expression des heterologen Gens über die Kombination CMV Enhancer/Hamster Ubiquitin/S27a Promotor gesteuert.

Der Vektor pBIN8a ist ein Derivat des Vektors pBIN und enthält ein modifiziertes NPT-Gen. Es handelt sich dabei um die NPT-Variante F2401 (Phe240Ile), deren Klonierung in WO2004/050884 beschrieben ist. In diesem Vektor wie auch in dem Vektor pBIDa, einem Derivat des Vektors pBID, steht die Expression des heterologen Gens unter der Kontrolle der CMV Enhancer/ Promotor-Kombination.

### ELISA (enzyme-linked immunosorbant assay)

Die Quantifizierung der exprimierten Antikörper (IgG1, IgG2 oder IgG4) in den Überständen von stabil transfizierten CHO-DG44 Zellen erfolgt mittels ELISA nach Standardprotokollen, wobei zum einen ein Ziege anti Human IgG Fc-Fragment (Dianova, Hamburg, DE) und zum anderen ein AP-konjugierter Ziege anti Human Kappa light chain Antikörper (Sigma) eingesetzt wird. Als Standard dient gereinigter Antikörper vom jeweils gleichen Isotyp wie die exprimierten Antikörper. Produktivitäten (pg/Zelle/Tag) werden dabei mit der Formel pg/((Ct-Co) t / In (Ct-Co)) berechnet, wobei Co und Ct die Zellzahl bei Aussaat bzw. Ernte und t die Kultivierungsdauer angibt.

### SEAP-Assay

Der SEAP-Titer in Kulturüberständen von transient transfizierten CHO-DG44 Zellen wird unter Verwendung des SEAP Reporter Gene Assays nach den Protokollvorgaben des Herstellers quantifiziert (Roche Diagnostics GmbH, Mannheim, DE).

### BEISPIEL 1: KLONIERUNG UND EXPRESSION VON IGG1 MIT C-TERMINALER LYSINDELETION

Die schwere Kette des monoklonalen humanisierten F19 Antikörpers (IgG1/kappa) wird als 1,5 kb Nael/Hindlll-Fragment aus dem Plasmid pG1D105F19HC (NAGENESEQ: AAZ32786) isoliert und in den mit EcoRI (mit Klenow-DNA-Polymerase aufgefüllt) und Hindlll-verdauten Vektor pBID kloniert, wobei der Vektor pBID/F19HC resultiert (Abb.1). Die leichte Kette hingegen wird als 1,3 kb HindIII/EcoRI-Fragment aus dem Plasmid pKN100F19LC (NAGENESEQ: AAZ32784) isoliert und in die entsprechenden Schnittstellen des Vektors pBIN kloniert, wodurch der Vektor pBIN/F19LC entsteht (Abb.1).

Die Deletion des C-terminalen Lysins an der schweren Kette des F19-Antikörpers erfolgt mittels PCR unter Verwendung des mutagenen Primers F19HC-Lys rev (SEQ ID NO:1) mit komplementärer Sequenz zur Gensequenz, die für die letzten Aminosäuren der schweren Kette im C-terminalen Bereich kodiert. Allerdings ist das Kodon des C-terminalen Lysins durch ein Stopp-Kodon ersetzt. Zudem schließt sich nachfolgend noch eine Xbal-Restriktionsschnittstelle an, die für die spätere Klonierung genutzt wird. Dieser mutagene Primer wird in Kombination mit dem Primer F19 heavy4 (SEQ ID NO:2) verwendet, der Komplementarität zu einer weiter stromaufwärts gelegenen Sequenz in der konstanten Region der schweren Kette aufweist. Als Template für die PCR-Mutagenese dient dabei der Vektor pBID/F19HC. Das resultierende PCR-Produkt von 757 bp wird mit BmgBI (einer stromabwärts von der Primerposition F19 heavy4 gelegenen endogenen Schnittstelle) und Xbal verdaut und das 547 bp Restriktionsfragment dazu verwendet, um den entsprechenden Sequenzbereich im Vektor pBID/F19HC zu ersetzen. Daraus resultiert der Vektor pBID/IgG1-Lys, der für eine schwere Kette des F19-Antikörpers mit deletierter C-terminaler Aminosäure Lysin kodiert (Abb. 1).

Durch transiente Transfektion von CHO-DG44 Zellen wird zunächst überprüft, ob das deletierte C-terminale Lysin, das bei allen IgG-Subtypen eine hoch konservierte Aminosäure ist, eine essentielle Bedeutung für die Expression oder Sekretion des IgG1-Moleküls hat. Es wird eine Co-Transfektion mit folgenden Plasmidkombinationen durchgeführt:
a) Kontrollplasmide pBID/F19HC und pBIN/F19LC, die für den monoklonalen Antikörper F19 in seiner Wildtypkonfiguration, also inklusive des C-terminalen Lysins an der schweren Kette, kodieren
b) pBID/1gGl-Lys und pBIN/F19LC, die für einen F19-Antikörper kodieren, dessen schwere Kette eine C-terminale Lysin-Deletion aufweist

Pro Kombination werden jeweils 10 Pools transfiziert, wobei in jeder Co-Transfektion äquimolare Mengen beider Plasmide eingesetzt werden. Nach 48 h Kultivierung in einem Gesamtvolumen von 3 mL erfolgt die Ernte und die Bestimmung des IgG1-Titers im Zellkulturüberstand mittels ELISA. Unterschiede in der Transfektionseffizienz werden durch Co-Transfektion mit einem SEAP-Expressionsplasmid (Zugabe von jeweils 100 ng Plasmid-DNA pro Transfektionsansatz) und nachfolgende Messung der SEAP-Aktivität korrigiert.

Überraschender Weise kann gezeigt werden, dass das IgG1-Molekül trotz der Deletion des C-terminalen Lysins in CHO-Zellen exprimiert und sezerniert wird und die Produktmengen vergleichbar mit der von IgG1 Wildtyp transfizierten Zellen sind (Abb. 2).

Für eine stabile Transfektion von CHO-DG44 Zellen wird eine Co-Transfektion mit den gleichen Plasmidkombinationen wie oben beschrieben durchgeführt, wobei pro Kombination jeweils 10 Pools erzeugt werden. Als Negativkontrolle werden 2 Mock-transfizierter Pools mitgeführt, d.h. gleich behandelt, aber ohne DNA-Zugabe im Transfektionsansatz. Die Selektion stabil transfizierter Zellen erfolgt zwei Tage nach der Transfektion in HT-freiem Medium mit Zusatz von 400 µg/mL G418. Nach erfolgter Selektion wird der IgG1-Titer und die spezifische Produktivität der Zellpools über einen Zeitraum von 3 - 4 Passagen ermittelt (Passagierungsrhythmus 2-2-3 Tage). Überraschender Weise zeigt sich, dass Zellen, die die Lysin-Deletionsvariante des IgG1 exprimieren, durchschnittlich sogar 27% höhere Titer bzw. 32% höhere spezifische Produktivitäten erzielen als Zellen, die den IgG1-Wildtyp exprimieren (Abb. 3). Dieser Produktionsvorteil der Lysin-Deletionsvariante ist auch dann noch gegeben, wenn in diesen Zellpools durch Zugabe von 100 nM MTX eine DHFR-basierte Genamplifikation induziert wird. Die Titer und spezifischen Produktivitäten sind durchschnittlich um 86% bzw. 120% höher (Abb. 4).

### BEISPIEL 2: KLONIERUNG UND EXPRESSION VON IGG4 MIT C-TERMINALER LYSINDELETION

Zur Expression eines monoklonalen humanisierten IgG4 Antikörpers (IgG4/kappa) wird die schwere Kette als 2,2 kb BamHI/SmaI-Fragment in das mit EcoRI (aufgefüllte Schnittstelle durch Behandlung mit Klenow-DNA-Polymerase)- und

BamHII-verdaute Plasmid pBIDa kloniert, wobei das Plasmid pBIDa/IgG4 HC resultiert (Abb. 1. Die leichte Kette hingegen wird als 1,1 kb BamHI/EcoRI-Fragment in die Schnittstellen BamHI/EcoRI des Plasmids pBINa kloniert, wodurch das Plasmid pBIN8a/IgG4 LC entsteht (Abb. 1).

Die Deletion des C-terminalen Lysins an der schweren Kette des IgG4-Antikörpers erfolgt mittels PCR unter Verwendung des mutagenen Primers IgG4HC-Lys rev (SEQ ID NO:3) mit komplementärer Sequenz zur Sequenz, die für die letzten Aminosäuren der schweren Kette im C-terminalen Bereich kodiert. Allerdings ist das Kodon des C-terminalen Lysins durch ein Stopp-Kodon ersetzt. Zudem schließt sich nachfolgend noch eine Xbal-Restriktionsschnittstelle an, die für die spätere Klonierung genutzt wird. Dieser mutagene Primer wird in Kombination mit dem Primer HC for8 (SEQ ID NO:4) verwendet, der Komplementarität zu einer weiter stromaufwärts gelegenen Sequenz in der konstanten Region der schweren Kette aufweist. Als Template für die PCR-Mutagenese dient dabei der Vektor pBIDa/IgG4 HC. Das resultierende PCR-Produkt von 1013 bp wird mit BmgBI (einer stromabwärts von der Primerposition HC for8 gelegenen endogenen Schnittstelle) und Xbal verdaut und das 644 bp Restriktionsfragment dazu verwendet, um den entsprechenden Sequenzbereich im Vektor pBIDa/IgG4 HC zu ersetzen. Daraus resultiert der Vektor pBIDa/IgG4-Lys, der für eine schwere Kette des F19-Antikörpers mit deletierter C-terminaler Aminosäure Lysin kodiert (Abb. 1).

Durch transiente Transfektion von CHO-DG44 Zellen wird zunächst überprüft, ob das deletierte C-terminale Lysin, das bei allen IgG-Subtypen eine hoch konservierte Aminosäure ist, eine essentielle Bedeutung für die Expression oder Sekretion des Moleküls hat. Es wird eine Co-Transfektion mit folgenden Plasmidkombinationen durchgeführt:
a) Kontrollplasmide pBIDa/IgG4 HC und pBIN8a/IgG4 LC, die für den monoklonalen IgG4 Antikörper in seiner Wildtypkonfiguration, also inklusive des C-terminalen Lysins an der schweren Kette, kodieren
b) pBIDa/IgG4-Lys und pBIN8a/IgG4 LC, die für einen monoklonalen IgG4-Antikörper kodieren, dessen schwere Kette eine C-terminale Lysin-Deletion aufweist
   Pro Kombination werden jeweils 10 Pools transfiziert, wobei in jeder Co-Transfektion äquimolare Mengen beider Plasmide eingesetzt werden. Nach 48 h Kultivierung in einem Gesamtvolumen von 3 mL erfolgt die Ernte und die Bestimmung des IgG4-Titers im Zellkulturüberstand mittels ELISA. Unterschiede in der Transfektionseffizienz werden durch Co-Transfektion mit einem SEAP-Expressionsplasmid (Zugabe von jeweils 100 ng Plasmid-DNA pro Transfektionsansatz) und nachfolgende Messung der SEAP-Aktivität korrigiert.

Überraschender Weise kann gezeigt werden, dass das IgG4-Molekül trotz der Deletion des C-terminalen Lysins in CHO-Zellen sogar etwas besser produziert wird als der IgG4 Wildtyp (Abb. 5).

Für eine stabile Transfektion von CHO-DG44 Zellen wird eine Co-Transfektion mit den gleichen Plasmidkombinationen wie oben beschrieben durchgeführt, wobei pro Kombination jeweils 10 Pools erzeugt werden. Als Negativkontrolle werden 2 Mock-transfizierter Pools mitgeführt, d.h. gleich behandelt, aber ohne DNA-Zugabe im Transfektionsansatz. Die Selektion stabil transfizierter Zellen erfolgt zwei Tage nach der Transfektion in HT-freiem Medium mit Zusatz von 400 µg/mL G418. Nach erfolgter Selektion wird durch Zugabe von 100 nM MTX eine DHFR-basierte Genamplifikation induziert wird. Die IgG4-Titer und die spezifische Produktivität der Zellpools werden über einen Zeitraum von 3 - 4 Passagen ermittelt (Passagierungsrhythmus 2-2-3 Tage). Insgesamt resultieren nach der Selektion und Amplifikation aus den mit IgG4 Wildtyp-transfizierten Zellen 4 und aus den mit der Lysin-Deletionsvariante transfizierten Zellen 6 stabil exprimierende Zellpools.. Überraschender Weise zeigt sich, dass Zellen, die die Lysin-Deletionsvariante des IgG4s exprimieren, durchschnittlich sogar 63 % höhere Titer bzw. 70 % höhere spezifische Produktivitäten erzielen als Zellen, die den IgG4-Wildtyp exprimieren (Abb. 6). Dieser Produktionsvorteil der Lysin-Deletionsvariante ist auch bei dem nachfolgenden Amplifikationsschritt mit 400 nM MTX gegeben. Hier werden im Durchschnitt 53% höhere Titer und 66% höhere spezifischen Produktivitäten erzielt (Abb. 7).

### BEISPIEL 3: KLONIERUNG UND EXPRESSION VON IGG2, IGG3, FC-FUSIONSPROTEINEN UND ANDEREN BIOMOLEKÜLEN MIT C-TERMINALER AMINOSÄUREDELETION

Zur Deletion des C-terminalen Lysins bei den schweren Ketten der Antikörper-Isotypen IgG2 und IgG3 wird die PCR-Mutagenese, wie schon in Beispiel 1 und 2 für die Isotypen 1 und 4 beschrieben, herangezogen. Auf gleiche Weise werden auch C-terminale Lysindeletionen an Fc-Fusionsproteinen (bivalent oder bispezifisch), bei denen Biomoleküle wie Zytokine, lösliche Rezeptoren etc. Bestandteil eines Fc-Fusionsproteins sind (Beispiele: Alefacept, LFA-3-Fc, Etanercept TNFR-Fc), vorgenommen.
Eine Erweiterung des Konzepts der Kodondeletion von C-terminalen Aminosäuren ist denkbar für Biomoleküle wie z.B. Erythropoetin (EPO) und tissue Plasminogen Activator (tPA), bei denen eine proteolytische Abspaltung des C-terminalen Arginins bekannt ist (M.A. Recny, H.A. Scoble and Y. Kim, J. Biol. Chem., 262 (1987) 17156-17163; Harris, R.J. (1995) Journal of Chromatography A, 705 (1), pp. 129-134). Voraussetzung dabei ist die Aufrechterhaltung der biologischen Aktivität oder wie z.B. im Fall von tPA die weiterhin intakte proteolytischen Prozessierung.
In transienten Transfektionen wird zunächst geprüft, ob die Deletion der C-terminalen Aminosäure einen Einfluss auf die Expression und Sekretion hat. Danach werden stabile Transfektionen durchgeführt und die spezifischen Produktivitäten und Titer von Zellpools, die mutierte Proteine oder Wildtyp-Proteine exprimieren, miteinander verglichen.

### BEISPIEL 4: REINIGUNG VON IGG1 WT UND IGG1 -LYS

Für den Isotyp IgG1 bzw. die WT- und die Lysin-Deletionsvariante ist die Aufarbeitung identisch. Die Protein A Affinitätschromatogrphie (MabSelect, GE) erfolgt gemäß den Angaben des Herstellers.
Die Quantifizierung der Produktausbeute nach ProteinA Chromatographie erfolgt mittels ProteinA HPLC. Die Ausbeuten liegen bei beiden Varianten des Isotyps IgG1 unabhängig von der Lysin-Kodondeletion bei über 90% (Abb. 8). Die Lysindeletion hat keinen negativen Einfluss auf die Affinitätschromatographie bzw. die Produktausbeute. Die Produktheterogenität bzgl. des C-terminalen Lysins wird sowohl qualitativ im isoelectric focusing (IEF) als auch quantitativ per weak cationic exchange (WCX) bestimmt (siehe Abb. 9 und 11). Um die durch C-terminales Lysin verursachte Ladungsheterogenität qualitativ mittel IEF zu bestimmen, werden die Antikörper mit Carboxypeptidase B inkubiert. Bei 37°C werden 10 µg Carboxypeptidase B in 100 µL und einer Antikörperkonzentration von 1 mg/mL für 2 h inkubiert. In Abb.9 zeigt sich für den WT-Antikörper (IgG1) nach enzymatischer Abspaltung mit Carboxypeptidase B (CpB) eine Reduktion in der Anzahl der Banden.

Die Kationaustauschchromatographie (ProPac WCX-10 / 4 x 250 mm) erfolgt mit einer Flussrate von 1 mL/min und einem Gradienten von 5-10% über 40 min (PufferA 20mM MES-Puffer pH 6,7; PufferB: 20mM , 1M NaCl pH6,7). Die Säule wird mit jeweils 40 µg Antikörper beladen. Der WT und die -Lys Variante werden jeweils mit bzw. ohne enzymatische CpB Behandlung analysiert.

Im Elutionsprofil bzw. Overlay des IgG1 WT sind die Zustände Lys1 und Lys2 durch die basischen Peaks 1 und 2 gekennzeichnet. Ihr Produktanteil beträgt ∼10 %. Das Elutionsprofil bzw. Overlay der Variante mit Lysindeletion weist eine sehr geringe Heterogenität im basischen Bereich auf. Der Produktanteil liegt unter 1 % (Abb.11).

### BEISPIEL 5: REINIGUNG VON IGG4 WT UND IGG4 -LYS

Für den Isotyp IgG4 bzw. die WT- und die Lysin-Deletionsvariante ist die Aufarbeitung identisch zum IgG1. Die Protein A Affinitätschromatogrphie (MabSelect, GE) erfolgt gemäß Angaben des Herstellers.
Die Quantifizierung der Produktausbeute nach ProteinA Chromatographie erfolgt mittels ProteinA HPLC. Die Ausbeuten liegen bei beiden Varianten des Isotyps IgG4 unabhängig von der Lysin-Kodondeletion ebenfalls bei über 90% (Abb. 8). Für den Isotyp IgG4 bzw. dessen Lysin-Codondeletion bestätigt sich wie bei IgG1 der nicht vorhandene negative Einfluss auf die Affinitätschromatographie bzw. die Produktausbeute. Die Produktheterogenität bzgl. des C-terminalen Lysins wird quantitativ per LC-MS bestimmt (siehe Abb. 10 und 12). Zur Bestimmung der C-terminalen Lysinverteilung werden die Antikörperproben zunächst mit DTT reduziert. Im Anschluss werden die reduzierte leichte Kette und die reduziete schwere Kette (HC 1-446 ohne Lys bzw. HC1-447 mit Lys) über HPSEC getrennt und in sich anschließender (in-line) ESI-TOF-MS analysiert. Die Verteilung des C-terminalen Lysins basiert auf den Peakflächen für die HC 1-446 bzw. HC 1-447. Im Massenspektrogramm der schweren Kette zeigt sich der Massenshift durch das Lysin in Abhängigkeit von der Glykosylierung (G0, G1, G2) (Abb. 12). Der ermittelte Produktanteil von Antikörpermolekülen (IgG4 WT) mit C-terminalen Lysinen in der schweren Kette (Lys1 und Lys2) beträgt ca. 20% (Abb. 10).

### BEISPIEL 6: THERMISCHE STABILITÄT

Die Bestimmung der thermischen Stabilität mittels intrinsischer Fluoreszenz (Tryptophan) zeigt keinen Einfluss des C-terminalen Lysins (IgG1 WT und -Lys Tₘ 69°C; bzw. IgG4 WT und -Lys 64°C). Die Anregungswellenlänge liegt bei 295 nm.

Das jeweilige Emissionsspektrum wird in 1°C Inkrementen über einen Bereich von 25°C bis 85°C vermessen. Die Emissionsspektren werden über einen Wellenlängenbereich von 300 nm bis 450 nm aufgenommen. Weitere technische Daten: Fluoreszenzspektrometer LS55 Perkin Elmer, Spaltbreite 4 nm für anregungs- und emissionsseitig, Temperaturmessung/PT1 00- in der Probe.

Die Proteinkonzentrationen betragen 0,1 mg/mL in PBS-Puffer. Die Untersuchung zeigt, dass das C-terminale Lysin der schweren Antikörperkette keinen Einfluss auf die thermische Stabilität des Antikörpermoleküls hat. Schon frühere Untersuchungen zeigten bereits, dass kein Einfluss des C-terminalen Lysins der schweren Antikörperkette auf die thermischen Stabilität des Antikörpermoleküle besteht (Liu et al. Immunol. Lett. 2006, 106 (2), 144-153).

## Patentansprüche

1. Verfahren zur Steigerung des Titers eines Proteins von Interesse einer Zelle **dadurch gekennzeichnet, dass**
a. in einer Nukleinsäuresequenz, die für das Protein von Interesse kodiert, mindestens das Kodon deletiert wird, das für die C-terminale Aminosäure kodiert,
b. die Zelle mit einem Vektor transfiziert wird, welcher die veränderte Nukleinsäure aus a) enthält und
c. die Zelle unter Bedingungen kultiviert wird, die eine Produktion des Proteins von Interesse erlaubt.

2. Verfahren gemäß Anspruch 1 **dadurch gekennzeichnet, dass** der Tlter um mindestens 10%, 20%, 50%, bevorzugt 75% gesteigert ist in Bezug auf den Vergleichswert des Proteins ohne die Deletion der C-terminalen Aminosäure.

3. Verfahren gemäß Anspruch 1 **dadurch gekennzeichnet, dass** die spezifische Produktivität der Zelle um mindestens 10%, 20%, 50%, bevorzugt 75% gesteigert ist in Bezug auf den Vergleichswert des Proteins ohne die Deletion der C-terminalen Aminosäure.

4. Verfahren zur Erzeugung eines Expressionsvektors zur gesteigerten Produktion eines Proteins von Interesse **dadurch gekennzeichnet, dass**
a. in der Nukleinsäuresequenz, die für das Protein von Interesse kodiert, mindestens das Kodon deletiert wird, das für die C-terminale Aminosäure kodiert, und
b. die so modifizierte Nukleinsäuresequenz aus a) in einen Expressionsvektor eingeführt wird.

5. Verfahren zur Erzeugung einer Zelle mit gesteigertem Titer eines Proteins von Interesse **dadurch gekennzeichnet, dass**
a. eine Gruppe von Zellen gemäß einem Verfahren nach Anspruch 1 behandelt wird und
b. anschließend eine Einzelzellklonierung vorgenommen wird.

6. Verfahren zur Herstellung eines Proteins von Interesse in einer Zelle
**dadurch gekennzeichnet, dass**
a. eine Gruppe von Zellen gemäß einem Verfahren nach Anspruch 1 behandelt wird,
b. diese Zellen aus a) in Anwesenheit zumindestens eines Selektionsdrucks selektioniert werden,
c. optional eine Einzelzellklonierung vorgenommen wird und
d. das Protein von Interesse aus den Zellen oder dem Kulturüberstand gewonnen wird.

7. Verfahren zur Herstellung mindestens eines Proteins von Interesse nach Anspruch 6 **dadurch gekennzeichnet, dass** die zur Herstellung verwendete Zellen nach Durchführung von Schritt b) zusätzlich einem Genamplifikationsschritt unterzogen werden.

8. Verfahren nach einem der Ansprüche 1 bis 7 **dadurch gekennzeichnet, dass** die C-terminale Aminosäure Lysin (Lys) oder Arginin (Arg) ist, bevorzugt Lys.

9. Verfahren nach einem der Ansprüche 1 bis 8 **dadurch gekennzeichnet, dass** das Protein von Interesse ein Antikörper, ein Fc-Fusionsprotein, EPO oder tPA ist.

10. Verfahren nach einem der Ansprüche 1 bis 7 **dadurch gekennzeichnet,**
**dass** das Protein von Interesse eine schwere Kette eines Antikörpers und die C-terminale Aminosäure Lysin (Lys) ist.

11. Verfahren nach Anspruch 10 **dadurch gekennzeichnet, dass** die schwere Kette des Antikörpers vom Typ IgG1, IgG2, IgG3 oder IgG4 ist, bevorzugt vom Typ IgG1, IgG2 oder IgG4.

12. Verfahren nach einem der Ansprüche 1 bis 11 **dadurch gekennzeichnet,**
**dass** das Protein von Interesse ein monoklonaler, polyklonaler, Säugetier-, Maus-, chimerer, humanisierter, Primaten- oder ein humaner Antikörper ist oder ein Antikörperfragment oder -derivat einer schweren Kette eines Immunoglobulin Antikörpers ist oder eines Fab, F(ab')2, Fc, Fc-Fc Fusionsproteins, Fv, single chain Fv, single domain Fv, tetravalenten single chain Fv, Disulfid-verknüpften Fv, Domänen-deletierten Antikörpers, eines Minibodys, Diabodys, oder eines Fusionspolypeptides eines der genannten Fragmente mit einem anderen Peptid oder Polypeptid ist oder ein Fc-Peptid Fusionsprotein, ein Fc-Toxin Fusionsprotein oder ein Scaffold-Protein.

13. Verfahren nach einem der Ansprüche 1 bis 12 **dadurch gekennzeichnet,**
**dass** die Zelle in Suspensionskultur kultiviert wird.

14. Verfahren nach einem der Ansprüche 1 bis 12 **dadurch gekennzeichnet,**
**dass** die Zelle serumfrei kultiviert wird.

15. Verfahren nach einem der Ansprüche 1 bis 14 **dadurch gekennzeichnet,**
**dass** die Zelle eine eukaryotische Zelle ist, z.B. aus Hefe, Pflanzen, Würmern, Insekten, Vögeln, Fischen, Reptilien oder Säugern.

16. Verfahren nach Anspruch 15 **dadurch gekennzeichnet, dass** die Zelle eine Säugerzelle ist.

17. Verfahren nach Anspruch 16 **dadurch gekennzeichnet, dass** die Zelle eine CHO Zelle ist, bevorzugt eine CHO DG44 Zelle.

18. Expressionsvektors mit gesteigerter Expression eines Gens von Interesse generierbar nach einem Verfahren gemäß Anspruch 4.

19. Zelle generierbar nach einem Verfahren gemäß Anspruch 5.

20. Verfahren zur Produktion und Reinigung eines Proteins von Interesse
**dadurch gekennzeichnet, dass**
a. in einer Nukleinsäuresequenz, die für das Protein von Interesse kodiert, mindestens das Kodon deletiert wird, das für die C-terminale Aminosäure kodiert, und
b. das erhaltene Protein von Interesse eine verringerte Heterogenität aufweist im Vergleich zum Protein ohne die Deletion der C-terminalen Aminosäure.

21. Verfahren nach Anspruch 20 **dadurch gekennzeichnet, dass** die C-terminale Aminosäure Lysin (Lys) oder Arginin (Arg) ist, bevorzugt Lys.

22. Verfahren nach einem der Ansprüche 20 oder 21 **dadurch gekennzeichnet,**
**dass** das Protein von Interesse ein Antikörper, ein Fc-Fusionsprotein, EPO oder tPA ist.

23. Verfahren nach einem der Ansprüche 20 bis 22 **dadurch gekennzeichnet,**
**dass** das Protein von Interesse eine schwere Kette eines Antikörpers ist und die C-terminale Aminosäure Lysin (Lys).

24. Verfahren nach Anspruch 23 **dadurch gekennzeichnet, dass** die schwere Kette des Antikörpers vom Typ IgG1, IgG2, IgG3 oder IgG4 ist, bevorzugt vom Typ IgG1, IgG2 oder IgG4.

25. Verfahren nach einem der Ansprüche 20 bis 24 **dadurch gekennzeichnet,**
**dass** bei der Reinigung des Proteins von Interesse eine niedrigere Salzkonzentration zum Einsatz kommt im Vergleich zur Aufreinigung eines Proteins ohne die Deletion der C-terminalen Aminosäure.


SEQUENCE LISTING

<110>  BOEHRINGER INGELHEIM PHARMA GMBH & CO KG

<120>  Verfahren zur Steigerung von Proteintitern

<130>  P01-2274

<160>  4

<170>  PatentIn version 3.3

<210>  1
<211>  33
<212>  DNA
<213>  Artificial

<220>
<223>  Primer F19HC-Lys rev

<400>  1
gacgtctaga tcaacccgga gacagggaga ggc                                33

<210>  2
<211>  22
<212>  DNA
<213>  Artificial

<220>
<223>  Primer F19 heavy 4

<400>  2
atctgcaacg tgaatcacaa gc                                              22

<210>  3
<211>  34
<212>  DNA
<213>  Artificial

<220>
<223>  Primer IgG4HC-Lys rev

<400>  3
gacgtctaga tcaacccaga gacagggaga ggct                                 34

<210>  4
<211>  20
<212>  DNA
<213>  Artificial

<220>
<223>  Primer HC for8

<400>  4
cccctgacct aagcccaccc                                                 20

SEQUENCE LISTING

<110>  BOEHRINGER INGELHEIM PHARMA GMBH & CO KG

<120>  Verfahren zur Steigerung von Proteintitern

<130>  P01-2274

<160>  4

<170>  PatentIn version 3.3

<210>  1
<211>  33
<212>  DNA
<213>  Artificial

<220>
<223>  Primer F19HC-Lys rev

<400>  1
gacgtctaga tcaacccgga gacagggaga ggc                                    33


<210>  2
<211>  22
<212>  DNA
<213>  Artificial

<220>
<223>  Primer F19 heavy 4

<400>  2
atctgcaacg tgaatcacaa gc                                                22


<210>  3
<211>  34
<212>  DNA
<213>  Artificial

<220>
<223>  Primer IgG4HC-Lys rev

<400>  3
gacgtctaga tcaacccaga gacagggaga ggct                                   34


<210>  4
<211>  20
<212>  DNA
<213>  Artificial

<220>
<223>  Primer HC for8

<400>  4
cccctgacct aagcccaccc                                                   20